# EUROPEAN PATENT APPLICATION

(11) **EP 4 589 036 A1**
(43) Date of publication of application: **23.07.2025**
(21) Application number: 23865565.8
(22) Date of filing: 13.09.2023
(51) Int. Cl.: C22B 3/28, B01D 11/04, C07C 43/04, C07C 59/125, C07C 211/09, C07C 229/26, C07C 237/06, C07C 237/12, C09K 3/00

(54) **METAL EXTRACTION AGENT, AND METHOD FOR SEPARATING AND RECOVERING METAL IONS USING SAID METAL EXTRACTION AGENT**

(30) Priority: 15.09.2022 JP 2022147027
(71) Applicant: FUJIFILM Corporation, Tokyo 106-8620 (JP)
(72) Inventor: KUSHIDA, Yo, Ashigarakami-gun, Kanagawa 258-8577 (JP); MOCHIZUKI, Hiroaki, Ashigarakami-gun, Kanagawa 258-8577 (JP)
(74) Representative: HGF
(86) International application number: PCT/JP2023/033379
(87) International publication number: WO 2024/058216

(57) **Abstract**

Provided are a metal extractant for extracting metal ions present in a water phase to an oil phase, and a separation recovery method of metal ions. In the metal extractant, nitrogen atoms positioned at both terminals of a molecular chain forming the metal extractant do not form a carbamoyl bond and include an unsubstituted hydrocarbon group and any coordinating functional group in a group G1 of coordinating functional groups below as a coordinating functional group (α) for metal ions to be extracted, or include an unsubstituted hydrocarbon group. The separation recovery method includes mixing an oil phase including the metal extractant and a water phase including plural kinds of metal ions.

## Description

### BACKGROUND OF THE INVENTION

### 1. Field of the Invention

The present invention relates to a metal extractant for extracting metal ions present in a water phase to an oil phase, and a separation recovery method of metal ions using this metal extractant.

### 2. Description of the Related Art

Metals that can be mined from mines are limited, and stable supply of noble metals required for precision equipment is a big challenge. Accordingly, it is important to recover valuable metals from industrial waste irrespective of mining.

In particular, along with the recent spread of electric vehicles, the amount of lithium ion batteries (LiB) wasted has been increasing every year. In LiB, a positive electrode active material including a metal element such as cobalt or nickel is used, and a significant increase in the demand for nickel is also expected. In order to deal with the increase in the demand for valuable metals along with the tidal current, not only an increase in the amount of mining but also a technique of recycling waste LiB into a metal are desired.

As a method of recycling waste into a metal, a wet extraction method (solvent extraction method) is used. In the wet extraction method, in a case where an aqueous solution (water phase) including ions of a metal element (simply referred to as metal ions) and an organic phase including a metal extractant are brought into contact with each other, mixed, and left to stand to separate the two phases, the metal ions to which the metal extractant is coordinated can be moved (extracted) to the organic phase. By extracting the organic phase, stripping the metal ions, and optionally purifying the metal ions, the waste can be recycled as a (high-purity) metal.

For example, JP2016-028021A describes a method of recovering metal ions using "a polyamine compound represented by Formula (1)" as a chelating agent (metal extractant). Specifically, JP2016-028021A describes that, as represented by Formula (1) below, copper ions in a water phase can be extracted to an oil phase using a polyamine compound where nitrogen atoms at terminals of a polyamine compound having an ethylenediamine skeleton are substituted with a carboxymethyl group and a substituted carbamoylmethyl group. In addition, JP2014-205900A describes "a method of removing impurities from a solution obtained by acid leaching of nickel oxide ores including valuable components such as nickel, cobalt, and scandium to separate the valuable components from the impurities" and "a specific valuable metal extractant consisting of an amide derivative represented by the following general formula (I)" used in this method. Specifically, this valuable metal extractant is an amide derivative where one amide group is introduced into an amino acid such as glycine as represented by Formula (I) described below.

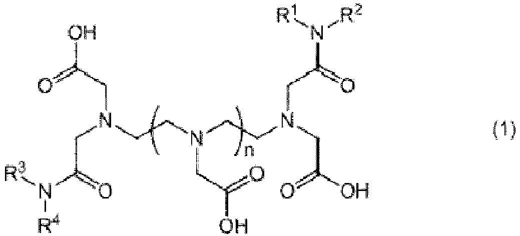

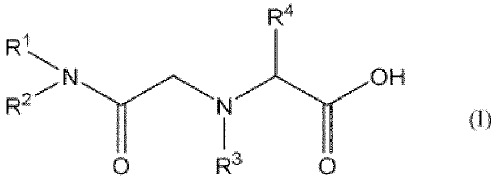

### SUMMARY OF THE INVENTION

JP2016-028021A and JP2014-205900A describe that, by using the metal extractants described in JP2016-028021A and JP2014-205900A, specific metal ions present in a water phase can be extracted and recovered to an oil phase. However, with any of the metal extractants, the selectivity or recovery rate of metal ions to be extracted is not sufficient, and there is room for improvement.

Further, JP2016-028021A and JP2014-205900A do not consider a configuration where, while extracting ions of two or more kinds of metal elements belonging to different groups in the periodic table of elements as ions of valuable metal elements among plural kinds of metal ions coexisting in a water phase, one kind of metal ions are recovered with high selectivity and high recovery rate (ions of two or more kinds of metal elements belonging to different groups are separated with high selectivity and high recovery rate). The reason for this is that, for example, needs for separating and recovering metal ions belonging to Groups 9 and 10 such as cobalt ions and nickel ions have rapidly increased due to recent rapid spread of lithium ion batteries, and it is not easy to separate and recover metal ions having similar physical behaviors and similar chemical behaviors in the related art. However, as long as one kind of metal ions can be recovered with high selectivity and high recovery rate while extracting metal ions (in particular, cobalt ions) belonging to Group 9 and metal ions (in particular, nickel ions) belonging to Group 10 having similar physical behaviors and similar chemical behaviors as ions of two or more kinds of valuable metal elements belonging to different groups, this configuration can largely contribute to further spread of electric vehicles and construction of a sustainable society.

An object of the present invention is to provide: a metal extractant that can extract specific metal ions from plural kinds of metal ions present in a water phase to an oil phase with high selectivity and high recovery rate; and a separation recovery method of metal ions using this metal extractant.

In addition, in one preferable aspect, an object of the present invention is to provide: a metal extractant that can extract one kind of metal ions to an oil phase with high selectivity and high recovery rate while extracting ions of two or more kinds of metal elements belonging to different groups in the periodic table among plural kinds of metal ions present in a water phase; and a separation recovery method of metal ions using this metal extractant.

The present inventors found that, regarding a metal extractant used in a wet extraction method of separating and recovering metal ions from a water phase including plural kinds of metal ions, by using a compound having a chemical structure where a molecular structure includes a molecular chain including nitrogen atoms at both terminals, the nitrogen atoms positioned at both terminals of the molecular chain do not form a carbamoyl bond, and a group including an unsubstituted hydrocarbon group and a specific coordinating functional group for metal ions to be extracted or two unsubstituted hydrocarbon groups are introduced into the nitrogen atoms, the specific metal ions can be extracted from a water phase to an oil phase with high selectivity and high recovery rate and can be separated and recovered from the other metal ions present in the water phase. In addition, by using the compound having the chemical structure as the metal extractant in the wet extraction method, while extracting ions of two or more kinds of metal elements belonging to different groups, particularly desirably, cobalt ions and nickel ions among plural kinds of metal ions present in the water phase, one kind of metal ions can be extracted to the oil phase with high selectivity and high recovery rate.

The present invention has been completed as a result of repeated investigation based on the above findings.

That is, the above-described objects have been achieved by the following means.
<1> A metal extractant for extracting metal ions present in a water phase to an oil phase,
   in which nitrogen atoms positioned at both terminals of a molecular chain forming the metal extractant do not form a carbamoyl bond and include a group including an unsubstituted hydrocarbon group and any coordinating functional group in a group G1 of coordinating functional groups below as a coordinating functional group (α) for metal ions to be extracted, or include an unsubstituted hydrocarbon group,
   <group G1 of coordinating functional groups>
   a hydroxyl group, an ether group, a thiol group, a thioether group, a nitrile group, an isonitrile group, a carboxy group, an amino group, an amide group, a phosphate group, a phosphonate group, a sulfonate group, a sulfinate group, a phosphino group, a nitrogen-containing heterocycle, an oxygen-containing heterocycle, and a sulfur-containing heterocycle.
<2> The metal extractant according to <1>,
   in which the group including the coordinating functional group is a hydrocarbon group including a coordinating functional group.
<3> The metal extractant according to <1> or <2>,
   in which at least one of the coordinating functional groups is selected from a carboxy group, a phosphate group, a phosphonate group, a sulfonate group, or a sulfinate group.
<4> The metal extractant according to any one of <1> to <3>, which is used for extracting and separating metal ions of two or more elements belonging to different groups in a periodic table among the metal ions.
<5> The metal extractant according to any one of <1> to <4>,
   in which the metal extractant is represented by Formula (I). 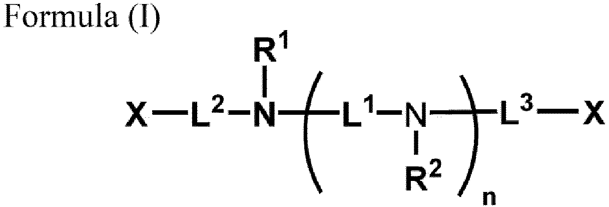 In Formula (I), R¹ represents an unsubstituted hydrocarbon group,
   R² represents a substituent,
   where, R² in N bonded to L³ represents an unsubstituted hydrocarbon group,
   X's each independently represent a group selected from a group G2 of coordinating functional groups below,
   L¹, L², and L³ each independently represent a linking group, and
   n represents an integer of 1 to 5,
   <group G2 of coordinating functional groups>
   a carboxy group, a phosphate group, a phosphonate group, a sulfonate group, a sulfinate group, and a hydroxyl group.
   <6> The metal extractant according to claim 5,
   in which X represents a carboxy group, a phosphate group, a phosphonate group, a sulfonate group, or a sulfinate group.
<7> The metal extractant according to <1>,
   in which the metal extractant is represented by Formula (II). 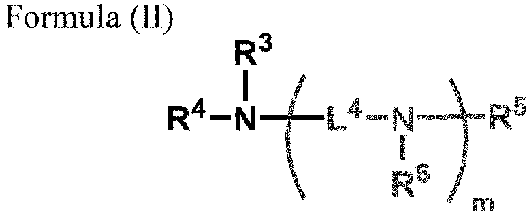 In Formula (II), R³ to R⁵ each independently represent an unsubstituted hydrocarbon group,
   R⁶ represents a substituent,
   where, R⁶ in N bonded to R⁵ represents an unsubstituted hydrocarbon group,
   L⁴ represents a linking group, and
   m represents an integer of 1 to 5.
<8> A separation recovery method of metal ions, the separation recovery method including:
   mixing a water phase including plural kinds of metal ions with an oil phase including the metal extractant according to any one of <1> to <7>.
<9> The separation recovery method according to <8>,
   in which in a case where the oil layer includes the metal extractant according to <7>, the water phase includes halide ions.
<10> The separation recovery method according to <8> or <9>,
   in which metal ions of two or more elements belonging to different groups in a periodic table among plural kinds of metal ions are extracted and recovered from the water phase to the oil phase.
<11> The separation recovery method according to <10>,
   in which the metal ions of the two or more elements are a metal recovery from a waste battery.

According to the present invention, it is possible to provide: a metal extractant that can extract specific metal ions from plural kinds of metal ions present in a water phase to an oil phase with high selectivity and high recovery rate; and a separation recovery method of metal ions using this metal extractant.

In addition, in one preferable aspect of the present invention, it is possible to provide: a metal extractant that can extract one kind of metal ions to an oil phase with high selectivity and high recovery rate while extracting ions of two or more kinds of metal elements belonging to different groups, particularly desirably, cobalt ions and nickel ions among plural kinds of metal ions present in a water phase; and a separation recovery method of metal ions using this metal extractant.

The above-described and other characteristics and advantageous effects of the present invention will be clarified from the following description appropriately with reference to the accompanying drawings.

### BRIEF DESCRIPTION OF THE DRAWINGS

Fig. 1 is a ¹H-NMR chart showing a metal extractant E-1 synthesized in Example.
Fig. 2 is an FT-IR chart showing the metal extractant E-1 synthesized in Example.
Fig. 3 is a ¹H-NMR chart showing a metal extractant E-3 synthesized in Example.

### DESCRIPTION OF THE PREFERRED EMBODIMENTS

In the present invention, in a case where a numerical range is shown to describe a content, physical properties, or the like of a component, any upper limit value and any lower limit value can be appropriately combined to obtain a specific numerical range in a case where an upper limit value and a lower limit value of the numerical range are described separately. In a case where a plurality of numerical ranges represented by "~" are set and described, the upper limit value and the lower limit value which form each of the numerical ranges are not limited to a specific combination described before and after "to" as a specific numerical range and can be set to a numerical range obtained by appropriately combining the upper limit value and the lower limit value of each numerical range. In the present invention, numerical ranges represented by "to" include numerical values before and after "to" as lower limit values and upper limit values.

**In** the present invention, the expression of a compound (for example, in a case where a compound is represented by an expression with "compound" added to the end) refers to not only the compound itself but also a salt or an ion thereof. **In** addition, this expression also refers to a derivative obtained by modifying a part of the compound, for example, by introducing a substituent into the compound within a range where the effects of the present invention do not deteriorate.

A substituent, a linking group, or the like (hereinafter, referred to as "substituent or the like") is not specified in the present invention regarding whether to be substituted or unsubstituted may have an appropriate substituent. Accordingly, even in a case where a YYY group is simply described in the present invention, this YYY group includes not only an aspect not having a substituent but also an aspect having a substituent. The same shall be applied to a compound which is not specified in the present specification regarding whether to be substituted or unsubstituted. Examples of a preferable substituent include groups selected from the substituent Z described below.

In the present invention, in a case where a plurality of substituents or the like represented by a specific reference numeral are present or a plurality of substituents or the like are simultaneously or alternatively defined, the respective substituents or the like may be the same as or different from each other. In addition, unless specified otherwise, in a case where a plurality of substituents or the like are adjacent to each other, the substituents may be linked or fused to each other to form a ring.

In the present specification, "metal elements belonging to different groups in the periodic table of elements" in the specific metal ion group will be referred to as "different-group metal elements", and particularly "different-group metal elements of the same period in the periodic table" will also be referred to as "same-period different-group metal elements". In addition, "ions of the different-group metal elements" and "ions of the same-period different-group metal elements" will also be referred to as "different-group metal ions" and "same-period different-group metal ions", respectively.

In the present invention, unless specified otherwise, "ppm" representing a content or the like is based on mass and represents "mass ppm".

### [Metal Extractant]

A metal extractant according to an embodiment of the present invention is a compound having a function of extracting metal ions present in a water phase to an oil phase, and can be suitably used particularly for a wet extraction method. By using the metal extractant according to the embodiment of the present invention in the wet extraction method, specific metal ions can be extracted from plural kinds of metal ions present in a water phase to an oil phase with high selectivity and high recovery rate. In addition, in a preferable aspect of the present invention, while extracting two or more kinds of different-group metal ions, for example, two or more kinds of metal ions belonging to Groups 4, 7, 9, and 10, particularly desirably, cobalt ions and nickel ions that are the same-period different-group metal ions as ions of valuable metal elements among plural kinds of metal ions present in a water phase, one kind of metal ions can be extracted to an oil phase with high selectivity and high recovery rate.

In the present invention, being capable of extracting metal ions with high selectivity represents that specific metal ions can be extracted and separated from the other metal ions such that, among the two or more kinds of extracted metal ions, a ratio of the amount of specific metal ions (typically one kind) to be extracted to the total amount of the other metal ions extracted [(the amount of the specific metal ions extracted)/(the total amount of the other metal ions extracted) is 3.0 or more (resolution, selection ratio). The ratio is preferably 3.5 or more, more preferably 5.0 or more, and particularly preferably 9.0 or more. The upper limit is not particularly limited and, in a case where two kinds of metal ions are extracted, can be, for example, 20.

In addition, in the present invention, being capable of extracting metal ions with high recovery rate represents that the metal ions can be extracted such that, regarding metal ions (specific metal ions to be extracted) extracted in the maximum amount the two or more kinds of extracted metal ions, a ratio of the amount of the metal ion extracted to the oil phase to the content of the metal ions (before the extraction) in the water phase [(the amount of the metal ions extracted to the oil phase)/(the content of the metal ions in the water phase] is 0.6 or more. The ratio is preferably 0.8 or more and more preferably 0.9 or more. The upper limit is not particularly limited and is ideally the total amount of the metal ions present in the water phase. For example, the upper limit is preferably 0.99 or less and can also be 0.95 or less or 0.90 or less. The specific amount of the metal ions extracted can be 30,000 mass ppm or less and preferably 20,000 mass ppm or less.

The detailed reason why the metal extractant according to the embodiment of the present invention exhibits the above-described effect is not yet clear but is presumed to be as follows. That is, in the metal extractant according to the embodiment of the present invention, nitrogen atoms at both terminals of the molecular chain do not form a carbamoyl bond. Therefore, it is considered that structural isomerization of complex ions formed by coordination to metal ions can be suppressed, and high selectivity can be obtained. In addition, it is considered that the terminal nitrogen atoms include an unsubstituted hydrocarbon group such that the dissolution stability of the metal extractant in the oil phase is improved, the extraction behavior of the metal ions in the oil phase is maintained to be fixed, and a high recovery rate can be obtained.

The metal extractant according to the embodiment of the present invention is a compound having a molecular structure shown in the following structure A.

Structure A: the molecular structure forming the metal extractant includes a molecular chain including nitrogen atoms at both terminals, and the nitrogen atoms positioned at both terminals of the molecular chain (hereinafter, also referred to as the terminal nitrogen atoms) do not form a carbamoyl bond and include a group including an unsubstituted hydrocarbon group and any coordinating functional group in a group G1 of coordinating functional groups below as a coordinating functional group (α) for metal ions to be extracted, or include an unsubstituted hydrocarbon group.

### <Group G1 of Coordinating Functional Groups>

a hydroxyl group, an ether group, a thiol group, a thioether group, a nitrile group, an isonitrile group, a carboxy group, an amino group, an amide group, a phosphate group, a phosphonate group, a sulfonate group, a sulfinate group, a phosphino group, a nitrogen-containing heterocycle, an oxygen-containing heterocycle, and a sulfur-containing heterocycle.

In one preferable aspect, the metal extractant according to the embodiment of the present invention is a compound having a molecular structure shown in the following structure B in addition to the structure A.

Structure B: a structure including any two or more coordinating functional groups in the group G1 of coordinating functional groups as coordinating functional groups (α) for metal ions to be extracted.

In the present invention, regarding the structure A, a metal extractant where the two terminal nitrogen atoms includes a group including one unsubstituted hydrocarbon group and one coordinating functional group (α) will be referred to as "metal extractant I", and a metal extractant where the two terminal nitrogen atoms includes two unsubstituted hydrocarbon groups will be referred to as "metal extractant II". In addition, in the present invention, unless specified otherwise, the metal extractant is a collective term including the metal extractant I and the metal extractant II.

The molecular chain including a nitrogen atom at both terminals in the structure A may be a straight chain, a branched chain, or a cyclic chain but is preferably a straight chain or a branched chain, in which a part of the straight chain or the branched chain may include a cyclic chain (cyclic structure) such as a cycloalkylene group or an arylene group. This molecular chain is preferably a straight chain not including a cyclic chain.

Here, the molecular chain is an atomic chain formed by bonding a plurality of atoms in a chain shape, and both terminals thereof are the longest chains that are nitrogen atoms. That is, the molecular chain refers to a chain (excluding a substituent bonded to the chain) where the groups bonded to the terminal nitrogen atoms are removed from the chemical structure of the metal extractant. For example, in Examples, a molecular chain of a metal extractant E-1 is N-CH₂-CH₂-N (ethylenediamine molecular chain), and a molecular chain of metal extractant T-1 is N-CO-CH₂-N-CH₂-CH₂-N-CH₂-CO-N (triethylenetetramine molecular chain).

The molecular chain is not particularly limited, and examples thereof include a chain including a linking chain L^{N} that bonds the terminal nitrogen atoms to each other. The linking group L^{N} is not particularly limited, and examples thereof include an alkylene group (preferably having 1 to 12 carbon atoms, more preferably 1 to 6 carbon atoms, and still more preferably having 1 to 4 carbon atoms), an alkenylene group (preferably having 2 to 6 carbon atoms and more preferably having 2 or 3 carbon atoms), an arylene group (having 6 to 24 carbon atoms and more preferably having 6 to 10 carbon atoms), an oxygen atom, a sulfur atom, an imino group (-NR^{N}-: R^{N} represents a hydrogen atom, an alkyl group having 1 to 6 carbon atoms or an aryl group having 6 to 10 carbon atoms), a carbonyl group, a phosphate linking group (-O-P(OH)(O)-O-), a phosphonate linking group (-P)(OH)(O)-O-), and a group including a combination thereof. The linking group L^{N} is preferably an alkylene group, an arylene group, a carbonyl group, an oxygen atom, a sulfur atom, an imino group, or a group including a combination thereof, and more preferably an alkylene group, an arylene group, an imino group, or a group including a combination thereof. The alkylene group and the alkenylene group may be a straight chain, a branched chain, or a cyclic chain but are preferably a straight chain or a branched chain, in which a part of the straight chain or the branched chain may include a cyclic chain (cyclic structure). The alkylene group and the alkenylene group are preferably a straight chain not including a cyclic chain and preferably a straight chain where the terminal nitrogen atoms are bonded to both terminals of the longest carbon chain. It is preferable that the linking chain L^{N} does not include a heteroatom, for example, an oxygen atom, a sulfur atom, or a nitrogen atom (imino group) at a terminal thereof, and it is more preferable that atoms at both terminals are carbon atoms.

In the group including the combination, the number of groups, linking groups, or atoms to be combined is not particularly limited and, for example, can be 2 to 15 and is preferably 2 to 10 and more preferably 2 to 5. In addition, the number of kinds of groups, linking groups, or atoms to be combined is not particularly limited and, for example, can be 2 or more and is preferably 2 or 3.

In particular, a hydrocarbon group or a linking group including an imino group is preferable, a hydrocarbon group or a group including a combination of a hydrocarbon group and an imino group is more preferable, and a hydrocarbon group is still more preferable. Here, examples of the hydrocarbon group include the alkylene group, the alkenylene group, and the arylene group. Among these, an alkylene group is preferable, and an ethylene group is more preferable. The group including a combination of a hydrocarbon group and an imino group is, for example, a group (polyamine skeleton) where a hydrocarbon group and an imino group are alternately bonded, and examples thereof include a -[hydrocarbon group-(imino group- hydrocarbon group)_{NN}]]- group. NN is not particularly limited, is an integer of 0 to 4, preferably an integer of 0 to 3 and more preferably 0 or 1.

The number of linking atoms in the linking chain L^{N} is preferably 15 or less, more preferably 13 or less, still more preferably 10 or less, and still more preferably 6 or less. The lower limit is 1 or more. The number of linking atoms refers to the minimum number of atoms that connect the terminal nitrogen atoms. The number of atoms forming the linking chain L^{N} is not uniquely determined depending on, for example, R^{N} having an imino group and can be appropriately set. For example, the number of atoms forming the linking chain L^{N} can be 1 to 100 and is preferably 1 to 40, more preferably 1 to 24, and still more preferably 1 to 12.

For example, in a case where the molecular chain is N-CH₂-CH₂-N, the number of atoms forming the linking chain L^{N} is 8, and the number of linking atoms is 4.

In the molecular chain, from the viewpoints of the selectivity and the recovery rate of metal ions, two terminal nitrogen atoms do not form a carbamoyl bond (also referred to as an amide bond which is a bond represented by -CO-NR^{N}-, in which R^{N} is as described above). In the present invention, the terminal nitrogen atoms not forming a carbamoyl group represents that, in a case where a carbon atom is directly bonded to the terminal nitrogen atom, this carbon atom does not include an oxo group (=O) as a substituent, and preferably represents that a carbon atom directly bonded to the terminal nitrogen atoms includes only a hydrogen atom, that is, is a methylene group.

In addition, in the metal extractant, regarding the group including the coordinating functional group (α) bonded to the terminal nitrogen atoms, it is preferable that the terminal nitrogen atoms do not form a carbamoyl bond. Further, another nitrogen atom (a nitrogen atom forming an amino group or an imino group) present in the molecular structure in addition to the terminal nitrogen atoms may form a carbamoyl group (-CO-N(R^{N})₂) or a carbamoyl bond. However, from the viewpoints of the selectivity and the recovery rate of metal ions, it is preferable that all of the nitrogen atoms do not form a carbamoyl group (-CO-N(R^{N})₂) and a carbamoyl bond.

The unsubstituted hydrocarbon group in the terminal nitrogen atoms is not particularly limited as long as it is a hydrocarbon group not including a substituent, and examples thereof include an unsubstituted alkyl group, an unsubstituted alkenyl group, an unsubstituted aryl group, an unsubstituted aralkyl group, and a combination thereof. Among these, an unsubstituted alkyl group or an unsubstituted aryl group is preferable, and an unsubstituted alkyl group is more preferable. The group including a combination is not particularly limited, and examples thereof include a group including a combination of an unsubstituted alkyl group or an unsubstituted aralkyl group and an unsubstituted aryl group. The unsubstituted hydrocarbon group does not include a coordinating functional group in the group G1 of coordinating functional groups described below.

The alkyl group forming the unsubstituted alkyl group, the unsubstituted alkenyl group, and the unsubstituted aralkyl group may be linear, branched, or cyclic and is preferably linear or branched and more preferably branched.

The number of carbon atoms in the unsubstituted alkyl group and the unsubstituted alkenyl group is not particularly limited and can be, for example, 1 to 36. From the viewpoint of solubility in the oil phase, the number of carbon atoms is preferably 4 to 30, more preferably 4 to 24, and still more preferably 6 to 20. The number of carbon atoms in the unsubstituted aryl group is not particularly limited, can be, for example, 6 to 24, and is preferably 6 to 10. The number of carbon atoms in the unsubstituted aralkyl group is not particularly limited, can be, for example, 7 to 25, and is preferably 7 to 11.

The group including the coordinating functional group may be a group including one or more coordinating functional groups in the group G1 of coordinating functional groups described below, and includes a group consisting of a coordinating functional group and a group consisting of a coordinating functional group and a binding group that bonds the coordinating functional group to the terminal nitrogen atoms.

The coordinating functional group will be described. The binding group is not particularly limited and has the same definition as that of L² in Formula (I) described below. Preferable examples of the binding group include a hydrocarbon group (an alkyl group, an alkenyl group, an aryl group, or a combination thereof) including a coordinating functional group.

The group including a coordinating functional group is preferably a group consisting of a coordinating functional group and a binding group that bonds the coordinating functional group to the terminal nitrogen atoms, and more preferably has the same definition as a "X-L²-" group in Formula (I) described below.

In the metal extractant I, combinations of unsubstituted alkyl groups and combinations of groups including a coordinating functional group in the terminal nitrogen atoms may be the same as or different from each other, respectively, but are preferably the same as each other.

In the metal extractant II, four unsubstituted alkyl groups in the terminal nitrogen atoms may be the same as or different from each other, it is preferable that one unsubstituted alkyl group in each of the terminal nitrogen atoms is the same as that of the other terminal nitrogen atom, and it is preferable that four unsubstituted alkyl groups are the same.

In one preferable aspect of the metal extractant according to the embodiment of the present invention, the molecular structure includes two or more coordinating functional groups (also simply referred to as functional groups) in the group G1 of coordinating functional groups below as coordinating functional groups (α) that are coordinate-bonded to the metal ions to be extracted (structure B).

The number of kinds of the coordinating functional groups in the metal extractant is not particularly limited and may be 1 or 2 or more. The number of kinds of the coordinating functional groups is preferably 1 to 5 and more preferably 1 or 2. In addition, the total number of coordinating functional groups in the metal extractant is at least two because the terminal nitrogen atom corresponds to an imino group as a coordinating functional group as described below. In a case where a coordinating functional group is provided at a site other than the terminal nitrogen atoms, the total number of coordinating functional groups in the metal extractant is not particularly limited as long as it is 3 or more, and is appropriately set. For example, in the metal extractant I, the total number of coordinating functional groups is preferably 4 or more, more preferably 4 to 10, still more preferably 4 to 8, and still more preferably 4 to 6. For example, in the metal extractant II, the total number of coordinating functional groups may be 2 or more and is preferably 2 to 8, more preferably 2 to 6, and still more preferably 2 to 4. In the metal extractant, the number of imino groups among the coordinating functional groups in the molecular chain is two or more and is appropriately determined in consideration of the total number of coordinating functional groups. For example, the number of imino groups is preferably 2 to 6 and preferably 2 or 3.

### <Group G1 of Coordinating Functional Groups>

a hydroxyl group (-OH), an ether group (-O-), a thiol group (-SH: a mercapto group; also referred to as a sulfanyl group), a thioether group (-S-), a nitrile group (-CN), an isonitrile group (-NC), a carboxy group (-COOH), an amino group (-N(R^{N})₂), an amide group (-CO-N(R^{N})₂: also referred to as a carbamoyl group), a phosphate group (-OPO₃H₂), a phosphonate group (-PO₃H₂), a sulfonate group (-SO₃H), a sulfinate group (-SO₂H), a phosphino group (-PH₂), a nitrogen-containing heterocycle, an oxygen-containing heterocycle, and a sulfur-containing heterocycle.

Here, R^{N} is as described above.

All of a phosphate group, a phosphonate group, a sulfonate group, and a sulfinate group include a group where at least one oxygen atom is substituted with a nitrogen atom or a sulfur atom.

Among the above-described coordinating functional groups, each of an amino group, an amide group, a phosphate group, a phosphonate group, a sulfonate group, a sulfinate group, a phosphino group, a nitrogen-containing heterocycle, an oxygen-containing heterocycle, a sulfur-containing heterocycle, and the like may be present as a substituent at the terminal of the molecular structure, or may be present as a linking group (bond) in the molecular structure, for example, in the molecular chain or the binding group described above. In this case, a hydrogen atom or a substituent in each of the groups or the rings is removed and introduced the molecular structure. For example, an amino group (-N(R^{N})₂) is introduced into the molecular structure as an imino group (-NR^{N}-). In the present invention, the amino group introduced into the molecular structure as an imino group corresponds to a coordinating functional group and is added to the number of coordinating functional groups in the metal extractant. Likewise, the terminal nitrogen atoms in the molecular chain correspond to the amino group as the coordinating functional group introduced as the imino group, and the number of two terminal nitrogen atoms in the molecular chain is added to the number of coordinating functional groups in the metal extractant.

In a case where an ether group or a thioether group is present at the terminal of the molecular chain, the ether group or the thioether group has a substituent. The substituent forming the ether group or the thioether group is not particularly limited. For example, a group selected from the substituent Z described below can be used, and preferable examples thereof include an alkyl group, an aryl group, and a heterocyclic group.

In a case where an amino group is introduced as a substituent, the amino group may form a carbamoyl group (-CO-N(R^{N})₂) but preferably does not form a carbamoyl group (a carbon atom directly bonded to the nitrogen atom does not include an oxo group as a substituent). On the other hand, in a case where an amino group is introduced into the molecular chain as an imino group, the imino group corresponding to the terminal nitrogen atoms does not form a carbamoyl bond (-CO-NR^{N}-), and the other imino group (also referred to as an internal nitrogen atom) may or may not form a carbamoyl bond (-CO-NR^{N}-). It is preferable that the internal nitrogen atom does not form a carbamoyl bond (-CO-NR^{N}-).

Each of a nitrogen-containing heterocycle, an oxygen-containing heterocycle, and a sulfur-containing heterocycle is a heterocycle including a carbon atom and at least one of a nitrogen atom, an oxygen atom, or a sulfur atom as ring-constituting atoms. The number of carbon atoms forming each of the heterocycles is not particularly limited and is, for example, preferably 2 to 20. Each of the heterocycles includes an aromatic heterocyclic ring and an aliphatic heterocyclic group, and a 5-membered ring or a 6-membered ring is preferable. Examples of each of the heterocycles include a heterocycle selected from the substituent Z described below.

An acidic group such as a carboxy group, a phosphate group, a phosphonate group, a sulfonate group, a sulfinate group, or a phosphino group, an amino group, an amide group, a nitrogen-containing heterocycle, or the like may form a salt. A cation that forms a salt is not particularly limited, and examples thereof include a metal cation, in particular, a metal cation belonging to Group 1 or Group 2, and an organic cation. The organic cation is not particularly limited, and examples thereof include an ammonium cation and an alkylammonium cation.

As the coordinating functional group in the metal extractant, among the functional groups belonging to the group G1 of coordinating functional groups, an amino group, a carboxy group, a phosphate group, a phosphonate group, a sulfonate group, a sulfinate group, a phosphino group, a hydroxyl group, or an ether group is preferable. As the coordinating functional group other than an amino group introduced into the molecular chain as an imino group among the coordinating functional groups, a carboxy group, a phosphate group, a phosphonate group, a sulfonate group, a sulfinate group, a hydroxyl group, or an ether group is more preferable, a carboxy group, a phosphate group, a phosphonate group, a sulfonate group, or a sulfinate group is still more preferable, and a carboxy group is still more preferable.

In a case where the metal extractant includes plural kinds of coordinating functional groups, a combination of the coordinating functional groups is not particularly limited, and the coordinating functional groups in the group G1 of coordinating functional groups can be appropriately combined with each other. For example, the metal extractant, in particular, the metal extractant I includes an amino group that is a coordinating functional group as an imino group (terminal nitrogen atom). Therefore, it is preferable that the metal extractant I includes an amino group that is the terminal nitrogen atom as a coordinating functional group and includes a functional group selected from a carboxy group, a phosphate group, a phosphonate group, a sulfonate group, a sulfinate group, or a hydroxyl group as at least one other coordinating functional group, it is preferable that the metal extractant I includes an amino group and at least one functional group selected from a carboxy group, a phosphate group, a phosphonate group, a sulfonate group, a sulfinate group, or an ether group, and it is more preferable that the metal extractant I includes an amino group and a carboxy group. On the other hand, the metal extractant II may include a coordinating functional group other than the amino group that is the terminal nitrogen atom but preferably does not include the other coordinating functional group.

As the metal extractant, a compound where the above-described unsubstituted hydrocarbon group or the like is introduced into terminal nitrogen atoms of mono or poly(alkylenediamine) corresponding to the above-described molecular chain is preferable, and a compound where the above-described unsubstituted hydrocarbon group or the like is introduced into terminal nitrogen atoms of mono or poly(ethylenediamine) is more preferable. Preferable examples of the mono or poly(alkylenediamine) include alkylenediamine, dialkylenetriamine, trialkylenetetramine, tetraalkylenepentamine, and pentaalkylenehexamine. Here, the alkylene group forming the mono or poly(alkylenediamine) has the same definition as the alkylene group forming the above-described linking chain L^{N}.

The metal extractant may include a substituent other than the coordinating functional groups in the group G1 of coordinating functional groups, and examples of the substituent that may be included in the metal extractant include groups selected from the substituent Z described below.

The metal extractant may have the above-described structures A and B. From the viewpoints of the selectivity and the recovery rate of metal ions, it is preferable that the metal extractant does not include a carbamoyl group and a carbamoyl bond.

The molecular weight of the metal extractant is not particularly limited, and can be, for example, 150 to 50,000. From the viewpoint of, for example, solubility in the oil phase, the molecular weight is preferably 200 to 10,000 and more preferably 250 to 1,000. In the present invention, in a case where the metal extractant is an oligomer, unless specified otherwise, the molecular weight of the oligomer refers to the number-average molecular weight in terms of standard polystyrene measured by gel permeation chromatography (GPC).

### -Measurement of Molecular Weight-

Regarding a method of measuring the molecular weight of the oligomer, basically, a value measured using a method under the following condition 1 or condition 2 (preferred) is used. In this case, an appropriate eluent may be selected and used depending on the kind of the oligomer.

### (Condition 1)

Column: Connect two TOSOH TSKgel Super AWM-H (product name, manufactured by Tosoh Corporation)
Carrier: 10 mM LiBr/N-methylpyrrolidone
Measurement temperature: 40°C
Carrier flow rate: 1.0 ml/min
Sample concentration: 0.1 mass%
Detector: refractive index (RI) detector

### (Condition 2)

Column: A column obtained by connecting TOSOH TSKgel Super HZM-H, TOSOH TSKgel Super HZ4000, and TOSOH TSKgel Super HZ2000 (all of which are trade names, manufactured by Tosoh Corporation)
Carrier: tetrahydrofuran
Measurement temperature: 40°C
Carrier flow rate: 1.0 ml/min
Sample concentration: 0.1 mass%
Detector: refractive index (RI) detector

An acid dissociation constant pKa of the metal extractant is not particularly limited, and an appropriate value for the kind, the number, and the like of the coordinating functional groups can be adopted. In the present invention, the pKa is a value measured by neutralization titration.

The metal extractant can be synthesized with reference to a well-known method, for example, the methods described in JP2016-028021A and JP2014-205900A. Examples of the method of synthesizing the metal extractant include synthesis methods described in Examples.

### <Metal Extractant I>

The metal extractant I among the metal extractants is preferably a compound represented by Formula (I) below.

The metal extractant I is a metal extractant including two to six nitrogen atoms as coordinating functional groups and two X's, in which the acid dissociation constant pKa is, for example, preferably 4 to 20, more preferably 5 to 15, and still more preferably 5 to 10.

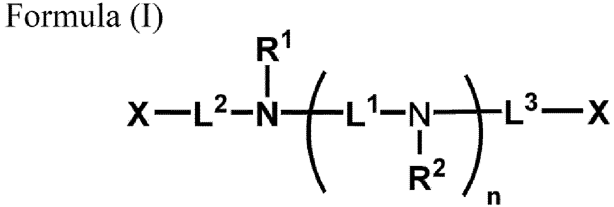

In Formula (I), R¹ represents an unsubstituted hydrocarbon group.

The unsubstituted hydrocarbon group that can be used as R¹ is not particularly limited, and has the same definition as the above-described unsubstituted hydrocarbon group in the terminal nitrogen atoms. In particular, an unsubstituted branched alkyl group having 4 to 20 carbon atoms is preferable.

In Formula (I), R² represents a substituent. The substituent that can be adopted as R² is not particularly limited. For example, a group selected from the substituent Z described below can be used, and preferable examples thereof include an alkyl group, an alkenyl group, an aralkyl group, an aryl group, and a heterocyclic group. In particular, from the viewpoint of solubility in the oil phase, a hydrocarbon group such as an alkyl group, an alkenyl group, an aralkyl group, an alkynyl group, or an aryl group is preferable. The hydrocarbon group that can be preferably used as R² is not particularly limited, and has the same definition as the above-described unsubstituted hydrocarbon group in the terminal nitrogen atoms. In addition, the hydrocarbon group may further include a group selected from the substituent Z. However, it is preferable that a carbon atom directly bonded to the nitrogen atom does not include an oxo group as a substituent, and it is more preferable that the hydrocarbon group is an unsubstituted hydrocarbon group.

Note that, in Formula (I), R² in N bonded to L³ among R²'s represents an unsubstituted hydrocarbon group. This unsubstituted hydrocarbon group has the same definition as the above-described unsubstituted hydrocarbon group in the terminal nitrogen atoms.

Each of the unsubstituted hydrocarbon group that can more preferably used as R² and the unsubstituted hydrocarbon group that can be used as R² in N bonded to L³ may be the same as or different from the unsubstituted hydrocarbon group that can be used as R¹, and are preferably the same as the unsubstituted hydrocarbon group that can be used as R¹.

In a case where the metal extractant I represented by Formula (I) includes a plurality of R²'s, the plurality of R²'s may be the same as or different from each other, but are preferably the same.

In Formula (I), X's each independently represent a coordinating functional group which is a group selected from the group G2 of coordinating functional groups below. X's each independently represent preferably a carboxy group, a phosphate group, a phosphonate group, a sulfonate group, or a sulfinate group and more preferably a carboxy group among the functional groups belonging to the group G2 of coordinating functional groups. Two X's may be the same as or different from each other, but are preferably the same. Each of the coordinating functional groups in the group G2 of coordinating functional groups has the same definition as each of the corresponding coordinating functional groups in the group G1 of coordinating functional groups below.

### <Group G2 of Coordinating Functional Groups>

a carboxy group, a phosphate group, a phosphonate group, a sulfonate group, a sulfinate group, and a hydroxyl group.

In Formula (I), L¹, L², and L³ each independently represent a linking group. The linking group that can be used as L¹, L², and L³ is not particularly limited, and examples thereof include an alkylene group, an alkenylene group (having preferably 2 to 6 carbon atoms and more preferably 2 or 3 carbon atoms), an arylene group (having preferably 6 to 24 carbon atoms and more preferably 6 to 10 carbon atoms), an oxygen atom, a sulfur atom, an imino group (-NR^{N}-: R^{N} has the same definition as R²), a carbonyl group, a phosphate linking group (-O-P(OH)(O)-O-), a phosphate linking group (-P(OH)(O)-O-), and a group including a combination thereof. The alkylene group and the alkenylene group may be a straight chain, a branched chain, or a cyclic chain but are preferably a straight chain or a branched chain, in which a part of the straight chain or the branched chain may include a cyclic chain (cyclic structure). The alkylene group and the alkenylene group are preferably a straight chain not including a cyclic chain and preferably a straight chain where the nitrogen atoms in Formula (I) are bonded to both terminals of the longest carbon chain. In the group including the combination, the number of groups, linking groups, or atoms to be combined is not particularly limited and, for example, can be 2 to 15 and is preferably 2 to 10 and more preferably 2 to 5. In addition, the number of kinds of groups, linking groups, or atoms to be combined is not particularly limited and, for example, can be 2 or more and is preferably 2 to 5 and more preferably 2 or 3.

As the linking group that can be used as L¹, among the above-described groups, an alkylene group is preferable, an alkylene group having 1 to 12 carbon atoms is preferable, an alkylene group having 1 to 6 carbon atoms is more preferable, and an alkylene group having 1 to 4 carbon atoms is still more preferable. The alkylene group that can be used as L¹ is preferably a linear alkylene group, more preferably a linear alkylene group where the nitrogen atoms in Formula (I) are bonded to both terminals of the longest carbon chain, and still more preferably an 1,2- ethanediyl group. The alkylene group that can be used as L¹ may include a substituent, and is preferably an unsubstituted alkylene group.

In a case where the metal extractant I represented by Formula (I) includes a plurality of L¹'s, the plurality of L¹'s may be the same as or different from each other, but are preferably the same.

In the linking group that can be used as L¹, a carbon atom directly bonded to the nitrogen atoms in Formula (I), in particular, the nitrogen atom in R¹ may include an oxo group as a substituent but preferably does not include an oxo group.

Among the above-described groups, an alkylene group is preferable as the linking group that can be used as L² and L³. The alkylene group that can be preferably used as L² and L³ may be linear, branched, or cyclic, and is preferably linear or branched, more preferably branched, and still more preferably a branched shape (1,1-alkanediyl group) where a carbon atom at one terminal is bonded to N and X in Formula (I) or a branched shape (1,2-alkanediyl group) where a carbon atom at one terminal and a carbon atom adjacent to the carbon atom are bonded to N and X in Formula (I). The number of carbon atoms in the alkylene group that can be used as L² and L³ is not particularly limited and can be, for example, 1 to 36. From the viewpoint of solubility in the oil phase, the number of carbon atoms in the alkylene group is preferably 4 to 30, more preferably 4 to 24, and still more preferably 6 to 20. The alkylene group that can be used as L² and L³ is more preferably an unsubstituted alkylene group.

**In** addition, in the linking group that can be used as L² and L³, a part of the carbon atoms forming the above-described alkylene group that can be preferably used as L² and L³ is substituted with an oxygen atom in one preferable aspect. **In** this aspect, the carbon atom substituted with the oxygen atom is a carbon atom positioned in the carbon chain forming the alkylene group, and the number of carbon atoms to be substituted is not particularly limited and can be 1 to 3. Examples of the group include a group (alkyl group-oxygen atom-alkyl group) where one carbon atom in the above-described alkylene group is substituted with an oxygen atom, and a group (alkylene group-oxygen atom-alkylene group-oxygen atom-alkyl group, for example, a metal extractant E-6 synthesized in Example) where two carbon atoms in the above-described alkylene group are substituted with an oxygen atom.

The linking groups that can be used as L² and L³ may be the same as or different from each other but are preferably the same. The linking group that can be used as L² and L³ may be the same as or different from a residue obtained by removing one hydrogen atom from the unsubstituted hydrocarbon group that can be used as R¹, but it is preferable that a group excluding the portion where X in Formula (I) and a nitrogen atom are bonded is the same as the unsubstituted hydrocarbon group that can be used as R¹.

In both of the linking groups that can be used as L² and L³, a carbon atom directly bonded to the nitrogen atoms in Formula (I) does not include an oxo group as a substituent.

In Formula (I), a group represented by -L²-X and a group represented by -L³-X are groups including a coordinating functional group, and are preferably a hydrocarbon group including a coordinating functional group, in which L², L³, and X are as described above. The hydrocarbon group including a coordinating functional group is not particularly limited and examples thereof include a group where a coordinating functional group is introduced into an alkyl group, an alkenyl group, an aryl group, or a group including a combination thereof. The alkyl group, the alkenyl group, and the aryl group correspond to the alkylene group, the alkenylene group, and the arylene group that can be used L², respectively, before introducing the coordinating functional group thereinto. The group including a combination is not particularly limited, and examples thereof include a group including a combination of an alkyl group and an aryl group.

In Formula (I), the group represented by -L²-X and a group represented by -L³-X may be the same as or different from each other and are preferably the same.

A combination of R¹, R², X and L¹ to L³ in Formula (I) is not particularly limited, and examples thereof include combinations of preferable examples of R¹, R², X and L¹ to L³.

In Formula (I), n represents an integer of 1 to 5, preferably an integer of 1 to 4, and more preferably 1 or 2.

The metal extractant I may include a substituent other than X, and examples of the substituent that may be included in the metal extractant I include groups selected from the substituent Z described below, which are groups other than the coordinating functional groups in the group G1 of coordinating functional groups.

Specific examples of the metal extractant I include the following compounds in addition to compounds synthesized in Examples, but the present invention is not limited thereto.

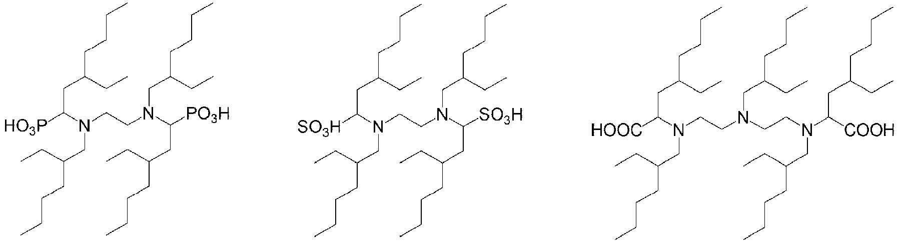

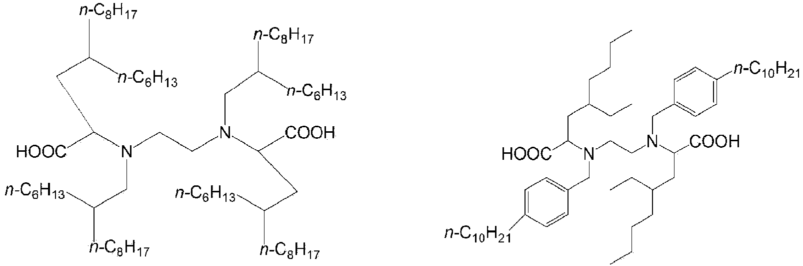

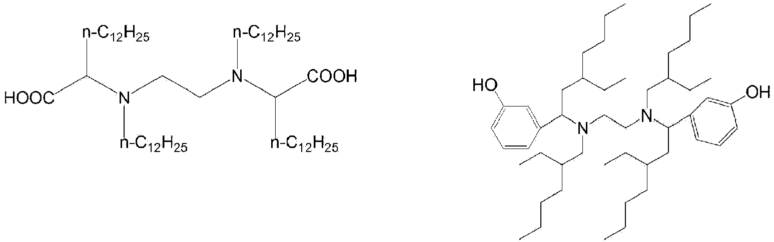

### <Metal Extractant II>

The metal extractant II among the metal extractants is preferably a compound represented by Formula (II) below.

The metal extractant II is a basic metal extractant including two to six nitrogen atoms (imino groups) as coordinating functional groups, in which the acid dissociation constant pKa of a conjugate acid is, for example, preferably 5.0 to 14.0 and more preferably 7.0 to 13.0. In the present invention, the pKa is a value measured by neutralization titration.

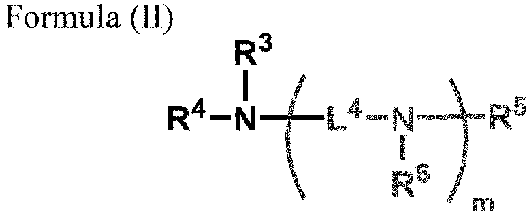

In Formula (II), R³ to R⁵ each independently represent an unsubstituted hydrocarbon group. The unsubstituted hydrocarbon group that can be used as R³ to R⁵ is not particularly limited, and has the same definition as the above-described unsubstituted hydrocarbon group in the terminal nitrogen atoms. In particular, an unsubstituted branched alkyl group having 4 to 20 carbon atoms is preferable. The unsubstituted hydrocarbon groups that can be used as R³ to R⁵ may be the same or may be different from each other but are preferably the same.

In R⁵*,* a carbon atom directly bonded to the nitrogen atom in R⁶ does not include an oxo group as a substituent.

In Formula (II), R⁶ represents a substituent. The substituent that can be adopted as R⁶ is not particularly limited. For example, a group selected from the substituent Z described below can be used, and preferable examples thereof include an alkyl group, an alkenyl group, an aralkyl group, an aryl group, and a heterocyclic group. In particular, from the viewpoint of solubility in the oil phase, a hydrocarbon group such as an alkyl group, an alkenyl group, an aralkyl group, an alkynyl group, or an aryl group is preferable. The hydrocarbon group that can be preferably used as R⁶ is not particularly limited, and has the same definition as the above-described unsubstituted hydrocarbon group in the terminal nitrogen atoms. In addition, the hydrocarbon group may further include a group selected from the substituent Z. However, it is preferable that a carbon atom directly bonded to the nitrogen atom does not include an oxo group as a substituent, and it is more preferable that the hydrocarbon group is an unsubstituted hydrocarbon group.

Note that, in Formula (II), R⁶ in N bonded to R⁵ among R⁶'s represents an unsubstituted hydrocarbon group. This unsubstituted hydrocarbon group has the same definition as the above-described unsubstituted hydrocarbon group in the terminal nitrogen atoms.

Each of the unsubstituted hydrocarbon group that can more preferably used as R⁶ and the unsubstituted hydrocarbon group that can be used as R⁶ in N bonded to R⁵ may be the same as or different from each of the unsubstituted hydrocarbon groups that can be used as R³ to R⁵. It is preferable that both of the unsubstituted hydrocarbon groups are the same, and it is preferable that both of the unsubstituted hydrocarbon groups are unsubstituted alkyl groups.

In a case where the metal extractant II represented by Formula (II) includes a plurality of R⁶'s, the plurality of R⁶'s may be the same as or different from each other, but are preferably the same.

In Formula (II), L⁴ represents a linking group. The linking group that can be used as L⁴ is not particularly limited, and examples thereof include an alkylene group (having preferably 1 to 12 carbon atoms, more preferably 1 to 6 carbon atoms, and still more preferably 1 to 4 carbon atoms), an alkenylene group (having preferably 2 to 6 carbon atoms and more preferably 2 or 3 carbon atoms), an arylene group (having preferably 6 to 24 carbon atoms and more preferably 6 to 10 carbon atoms), an oxygen atom, a sulfur atom, an imino group (-NR^{N}-: R^{N} has the same definition as R²), a carbonyl group, a phosphate linking group (-O-P(OH)(O)-O-), a phosphate linking group (-P(OH)(O)-O-), and a group including a combination thereof. The alkylene group and the alkenylene group may be a straight chain, a branched chain, or a cyclic chain but are preferably a straight chain or a branched chain, in which a part of the straight chain or the branched chain may include a cyclic chain (cyclic structure). The alkylene group and the alkenylene group are preferably a straight chain not including a cyclic chain and preferably a straight chain where the nitrogen atoms in Formula (I) are bonded to both terminals of the longest carbon chain. In the group including the combination, the number of groups, linking groups, or atoms to be combined is not particularly limited and, for example, can be 2 to 15 and is preferably 2 to 10 and more preferably 2 to 5. In addition, the number of kinds of groups, linking groups, or atoms to be combined is not particularly limited and, for example, can be 2 or more and is preferably 2 or 3.

Among the above-described groups, the linking group that can be used as L⁴ is preferably a linear alkylene group, more preferably a linear alkylene group where the nitrogen atoms in Formula (II) are bonded to both terminals of the longest carbon chain, and still more preferably an 1,2- ethanediyl group. The alkylene group that can be used as L⁴ may include a substituent, and is preferably an unsubstituted alkylene group.

In a case where the metal extractant II represented by Formula (II) includes a plurality of L⁴'s, the plurality of L⁴'s may be the same as or different from each other, but are preferably the same.

In the linking group that can be used as L⁴, a carbon atom directly bonded to the nitrogen atoms in R³ and R⁴ does not include an oxo group as a substituent. However, a carbon atom directly bonded to the nitrogen atom in R⁶ may include an oxo group as a substituent but preferably does not include an oxo group.

In Formula (II), the amino group in R³ and R⁴ and the amino group in R⁵ and R⁶ may be the same as or different from each other and are preferably the same.

A combination of R³ to R⁶ and L⁴ in Formula (II) is not particularly limited, and examples thereof include combinations of preferable examples of R³ to R⁶ and L⁴.

In Formula (II), m represents an integer of 1 to 5, preferably an integer of 1 to 4, and more preferably 1 or 2.

The metal extractant II may include a substituent, and examples of the substituent that may be included in the metal extractant II include groups selected from the substituent Z described below.

Specific examples of the metal extractant II include the following compounds in addition to compounds synthesized in Examples, but the present invention is not limited thereto.

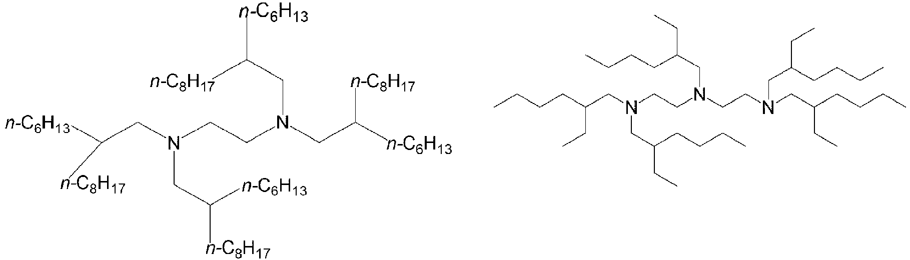

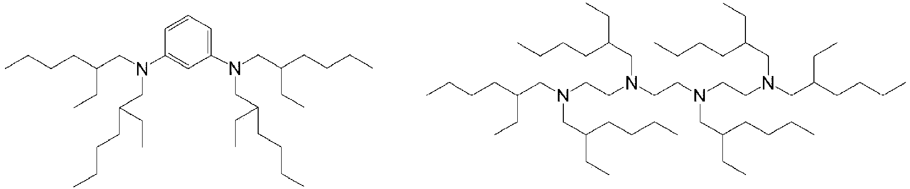

### -Substituent Z-

The substituent Z includes: an alkyl group (preferably an alkyl group having 1 to 20 carbon atoms, for example, methyl, ethyl, isopropyl, t-butyl, pentyl, heptyl, 1-ethylheptyl, benzyl, 2-ethoxyethyl, or 1-carboxymethyl); an alkenyl group (preferably an alkenyl group having 2 to 20 carbon atoms, for example, vinyl, allyl, or oleyl); an alkynyl group (preferably an alkynyl group having 2 to 20 carbon atoms, for example, ethynyl, butadiynyl, or phenyl-ethynyl); a cycloalkyl group (preferably a cycloalkyl group having 3 to 20 carbon atoms; for example, cyclopropyl, cyclopentyl, cyclohexyl, or 4-methylcyclohexyl; the meaning of an alkyl group described in the present invention typically includes a cycloalkyl group but, here, an alkyl group and a cycloalkyl group are distinguished from each other); an aryl group (preferably an aryl group having 6 to 26 carbon atoms, for example, phenyl, 1-naphthyl, 4-methoxyphenyl, 2-chlorophenyl, or 3-methylphenyl); an aralkyl group (preferably an aralkyl group having 7 to 23 carbon atoms, for example, benzyl or phenethyl); a heterocyclic group (preferably a heterocyclic group having 2 to 20 carbon atoms and more preferably a 5- or 6-membered heterocyclic group having at least one oxygen atom, sulfur atom, or nitrogen atom; the heterocyclic group includes an aromatic heterocyclic group and an aliphatic heterocyclic group; for example, a tetrahydropyran ring group, a tetrahydrofuran ring group, 2-pyridyl, 4-pyridyl, 2-imidazolyl, 2-benzimidazolyl, 2-thiazolyl, 2-oxazolyl, a pyrrolidone group); an alkoxy group (preferably an alkoxy group having 1 to 20 carbon atoms, for example, methoxy, ethoxy, isopropyloxy, or benzyloxy); an aryloxy group (preferably an aryloxy group having 6 to 26 carbon atoms, for example, phenoxy, 1-naphthyloxy, 3-methylphenoxy, or 4-methoxyphenoxy); a heterocyclic oxy group (a group in which an -O-group is bonded to the above-described heterocyclic group); an alkoxycarbonyl group (preferably an alkoxycarbonyl group having 2 to 20 carbon atoms, for example, ethoxycarbonyl, 2-ethylhexyloxycarbonyl, or dodecyloxycarbonyl); an aryloxycarbonyl group (preferably an aryloxycarbonyl group having 7 to 26 carbon atoms, for example, phenoxycarbonyl, 1-naphthyloxycarbonyl, 3-methylphenoxycarbonyl, or 4-methoxyphenoxycarbonyl); a heterocyclic oxycarbonyl group (a group in which an -O-CO-group is bonded to the above-described heterocyclic group); an amino group (preferably an amino group having 0 to 20 carbon atoms, an alkylamino group, or an arylamino group, for example, amino (-NH₂), N,N-dimethylamino, N,N-diethylamino, N-ethylamino, or anilino); a sulfamoyl group (preferably a sulfamoyl group having 0 to 20 carbon atoms, for example, N,N-dimethylsulfamoyl or N-phenylsufamoyl); an acyl group (an alkylcarbonyl group, an alkenylcarbonyl group, an alkynylcarbonyl group, an arylcarbonyl group, or a heterocyclic carbonyl group, preferably an acyl group having 1 to 20 carbon atoms, for example, acetyl, propionyl, butyryl, octanoyl, hexadecanoyl, acryloyl, methacryloyl, crotonoyl, benzoyl, naphthoyl, or nicotinoyl); an acyloxy group (an alkylcarbonyloxy group, an alkenylcarbonyloxy group, an alkynylcarbonyloxy group, or a heterocyclic carbonyloxy group, preferably an acyloxy group having 1 to 20 carbon atoms, for example, acetyloxy, propionyloxy, butyryloxy, octanoyloxy, hexadecanoyloxy, acryloyloxy, methacryloyloxy, crotonoyloxy, or nicotinoyloxy); an aryloyloxy group (preferably an aryloyloxy group having 7 to 23 carbon atoms, for example, benzoyloxy or naphthoyloxy); a carbamoyl group (preferably a carbamoyl group having 1 to 20 carbon atoms, for example, N,N-dimethylcarbamoyl or N-phenylcarbamoyl); an acylamino group (preferably an acylamino group having 1 to 20 carbon atoms, for example, acetylamino or benzoylamino); an alkylthio group (preferably an alkylthio group having 1 to 20 carbon atoms, for example, methylthio, ethylthio, isopropylthio, or benzylthio); an arylthio group (preferably an arylthio group having 6 to 26 carbon atoms, for example, phenylthio, 1-naphthylthio, 3-methylphenylthio, or 4-methoxyphenylthio); a heterocyclic thio group (a group in which an -S- group is bonded to the above-described heterocyclic group); an alkylsulfonyl group (preferably an alkylsulfonyl group having 1 to 20 carbon atoms, for example, methylsulfonyl or ethylsulfonyl); an arylsulfonyl group (preferably an arylsulfonyl group having 6 to 22 carbon atoms, for example, benzenesulfonyl); an alkylsilyl group (preferably an alkylsilyl group having 1 to 20 carbon atoms, for example, monomethylsilyl, dimethylsilyl, trimethylsilyl, or triethylsilyl); an arylsilyl group (preferably an arylsilyl group having 6 to 42 carbon atoms, for example, triphenylsilyl); an alkoxysilyl group (preferably an alkoxysilyl group having 1 to 20 carbon atoms, for example, monomethoxysilyl, dimethoxysilyl, trimethoxysilyl, or triethoxysilyl); an aryloxysilyl group (preferably an aryloxysilyl group having 6 to 42 carbon atoms, for example, triphenyloxysilyl); a phosphoryl group (preferably a phosphate group having 0 to 20 carbon atoms, for example, -OP(=O)(R^{P})₂); a phosphonyl group (preferably a phosphonyl group having 0 to 20 carbon atoms, for example, -P(=O)(R^{P})₂); a phosphinyl group (preferably a phosphinyl group having 0 to 20 carbon atoms, for example, -P(R^{P})₂); a phosphonate group (preferably a phosphonate group having 0 to 20 carbon atoms, for example, -PO(OR^{P})₂); a sulfo group (sulfonate group); a carboxy group; a hydroxy group; a sulfanyl group; a cyano group; a halogen atom (for example, a fluorine atom, a chlorine atom, a bromine atom, or an iodine atom). R^{P} represents a hydrogen atom or a substituent (preferably a group selected from the substituent Z).

In addition, each group exemplified in the substituent Z may be further substituted with the substituent Z.

The alkyl group, the alkylene group, the alkenyl group, the alkenylene group, the alkynyl group, the alkynylene group, and/or the like may be cyclic or chained, may be linear or branched.

### [Separation Recovery Method of Metal Ions]

A separation recovery method of metal ions according to an embodiment of the present invention (hereinafter, also referred to as the separation recovery method according to the embodiment of the present invention) is a method of mixing a water phase including plural kinds of metal ions and an oil phase including the metal extractant according to the embodiment of the present invention. As a result, the specific metal ions to which the metal extractant according to the embodiment of the present invention is coordinated can be moved (extracted) to be separated and recovered from the water phase to the oil phase with high selectivity and high recovery rate. Here, the metal ions to be extracted to the oil phase may be a part of the plural kinds of metal ions in the water phase, which is two or more kinds of metal ions, or may be all kinds of different-group metal ions in the water phase. **In** the separation recovery method according to the embodiment of the present invention, one kind of metal ions can be extracted to an oil phase with high selectivity and high recovery rate as ions of a valuable metal elements among the two or more kinds of different-group metal ions, for example, two or more kinds of metal ions belonging to Groups 4, 7, 9, and 10, particularly desirably, cobalt ions and nickel ions that are the same-period different-group metal ions.

The present inventors found that the metal extractant according to the embodiment of the present invention has the property and function in which, while extracting two or more kinds of metal ions among plural kinds of metal ions present in the water phase to the oil phase together, one kind of metal ions can be extracted to the oil phase with high selectivity and high recovery rate, and applies the separation recovery method according to the embodiment of the present invention to the new use where two or more kinds of different-group metal ions are separated and recovered.

### <Water Phase>

Water formed in the water phase is not particularly limited, and (super) pure water, ion exchange water, or the like can be used.

The metal ions in the water phase only need to include at least one kind of metal ions belonging to Group 3 to Group 16, and may include metal ions belonging to groups other than Group 3 to Group 16. In the present invention, it is preferable that two or more kinds of metal ions belonging to Group 3 to Group 16 are included, it is more preferable that ions of at least one kind of a transition metal element (transition metal element belonging to Group 3 to Group 12), it is still more preferable that two or more kinds of metal ions belonging to Group 4 to Group 12 are included, it is still more preferable that two or more kinds of metal ions belonging to Group 4 to Group 10 are included, it is still more preferable that two or more kinds of metal ions belonging to Groups 4, 7, 9, and 10 are included, and it is most preferable that two or more kinds of metal ions belonging to Group 8 to Group 10 are included. The metal ions belonging to each of the groups are not particularly limited, metal ions belonging to the fourth to sixth periods in the periodic table are preferable, and metal ions belonging to the fourth period or the fifth period are more preferable. In addition, the number of kinds of the metal ions is not particularly limited as long as it is 2 or more. For example, the number of kinds of the metal ions can be 2 to 15 and is preferably 2 to 8 and more preferably 2 to 5.

A combination of the plurality of metal ions is not particularly limited, and examples of a combination of groups include a combination including Group 9 and Group 10 (excluding a combination including Group 7), a combination including Group 4 and Group 9, a combination including Group 7, Group 9, and Group 10, and a combination of Group 7, Group 8, Group 9, and Group 10.

In the present invention, the number of kinds of metal ions belonging to each of the groups may be two or more but, from the viewpoint of exhibiting high selectivity, is preferably one.

Specific examples of the combination of the metal ions include a combination including Co and Ni (excluding a combination including Mn), a combination including Zr and Rh, a combination including Mn, Co, and Ni and a combination including Mn, Fe, Co, and Ni.

The metal element belonging to each of the groups is not particularly limited, and an appropriate atom can be used. Preferable examples of a metal element belonging to Group 3 include Sc and Y.

Preferable examples of a metal element belonging to Group 4 include Ti, Zr, and Hf.

Preferable examples of a metal element belonging to Group 5 include V, Nb, and Ta.

Preferable examples of a metal element belonging to Group 6 include Cr, Mo, and W.

Preferable examples of a metal element belonging to Group 7 include Mn and Tc.

Preferable examples of a metal element belonging to Group 8 include Fe, Ru, and Os.

Preferable examples of a metal element belonging to Group 9 include Co, Rh, and Ir.

Preferable examples of a metal element belonging to Group 10 include Ni, Pd, and Pt.

Preferable examples of a metal element belonging to Group 11 include Cu, Ag, and Au.

Preferable examples of a metal element belonging to Group 12 include Zn, Cd, and Hg.

Preferable examples of a metal element belonging to Group 13 include Al, Ga, In, and Tl.

Preferable examples of a metal element belonging to Group 14 include Ge, Sn, and Pb.

Preferable examples of a metal element belonging to Group 15 include Sb and Bi.

Preferable examples of a metal element belonging to Group 16 include Te.

The plural kinds of metal ions can be appropriately prepared and, for example, various metal salts (salts of typical elements with inorganic acids such as nitric acid or sulfuric acid or organic acids such as acetic acid), a mixture of mined metals (ion), a recovery from metal waste, other waste such as a metal recovery from a waste battery (LiB), or a mixture thereof can be used. Examples of the metal recovery from the waste LiB include recoveries obtained using a well-known method such as a wet process or electrolysis.

A total content of the plural kinds of metal ions in the water phase is not particularly limited and is appropriately set. For example, the total content can be 1,000 to 1,000,000 mass ppm and is preferably 1,000 to 100,000 mass ppm and more preferably 1,000 to 80,000 mass ppm.

A total content of the metal ions belonging to Groups 8 to 12 among the metal ions is not particularly limited and is appropriately set. For example, the content can be 1,000 to 80,000 mass ppm and is preferably 1,000 to 60,000 mass ppm.

A total content of the metal ions belonging to Group 3 to Group 7 and Group 13 to Group 16 among the metal ions is not particularly limited and is appropriately set. For example, the total content can be 1,000 to 60,000 mass ppm and is preferably 1,000 to 30,000 mass ppm.

A content of the metal ions belonging to each of the groups is not particularly limited and is appropriately set. For example, the content can be 1,000 to 60,000 mass ppm and is preferably 1,000 to 50,000 mass ppm.

In a case where metal ions belonging to groups other than Group 3 to Group 16 are included, the total content thereof is not particularly limited and can be preferably 50,000 mass ppm or less and is preferably 30,000 mass ppm or less.

In a case where two or more kinds of metal ions belonging to each of the groups are present, the content of the metal ions belonging to each of the groups is the total content.

In the present invention, the content of the metal ions belonging to one group may be more than or less than a content of the metal ions belonging to another group. In the separation recovery method according to the embodiment of the present invention, the metal ions can be separated and recovered with high selectivity. Therefore, the contents of the metal ions belonging to different groups do not need to be set at a specific ratio. For example, a mass ratio of the content of metal ions belonging to a specific group (for example, metal ions extracted in the maximum amount) to the content of metal ions belonging to another group (for example, metal ions other than the metal ions extracted in the maximum amount (including metal ions that are not extracted) [the content of the metal ions belonging to the specific group:the content of the metal ions belonging to the other group] can be, for example, 100:1 to 10,000 and is preferably 100:10 to 5,000, more preferably 100:50 to 1,000, and still more preferably 100:70 to 130.

The pH of the water phase is not particularly limited and is appropriately set. For example, the pH of the water phase is preferably 0.1 to 10 in consideration of the solubility of the metal ions, the formation of complex ions, and the like. The pH of the water phase can be adjusted, for example, using an acid or an alkali. As the acid, a well-known acid can be used without any particular limitation, and examples thereof include an inorganic acid such as sulfuric acid, hydrochloric acid, nitric acid, or phosphoric acid and an organic acid such as formic acid, acetic acid, oxalic acid, organic phosphoric acid, or organic sulfonic acid. As the alkali, a well-known alkali can be used without any particular limitation, and examples thereof include an inorganic alkali and an organic alkali. Among these, an inorganic alkali is preferable. Examples of the inorganic alkali include a hydroxide of a metal belonging to Group 1 or Group 2, a metal alkali such as a carbonate, ammonia water, and ammonium chloride. Examples of the organic alkali include an organic ammonium salt.

The temperature of the water phase is not particularly limited and can be, for example, 10°C to 60°C.

The water phase may optionally include, for example, a ligand coordinated to metal ions or a compound that generates the ligand.

The water phase can be prepared by dissolving metal ions in water. Preparation conditions of the water phase are not particularly limited. For example, the preparation temperature can be 10°C to 60°C.

The water phase may include a masking agent in addition to the above-described metal ions. As the masking agent, various well-known agents can be used without any particular limitation. Examples of the masking agent include a monodentate ligand such as ammonia or a chelating agent such as dithizone.

### <Oil Phase>

In the separation recovery method according to the embodiment of the present invention, the oil phase (organic phase) including one kind or two or more kinds of the metal extractants according to the embodiment of the present invention is used for the above-described water phase.

The metal extractant according to the embodiment of the present invention exhibits solubility in an organic solvent, is present in the oil phase, is coordinate-bonded to two or more kinds of metal ions present in the vicinity of an interface between the water phase and the oil phase, and has a function of moving the two or more kinds of metal ions to the oil phase. In the present invention, the solubility in the organic solvent refers to a property in which the metal extractant is soluble in the organic solvent in a content described below.

The organic solvent for forming the oil phase is not particularly limited, and an appropriate organic solvent can be used. Examples of the organic solvent include an alcohol solvent, an ether solvent, a hydrocarbon-based solvent (an aromatic solvent or an aliphatic solvent), and a halogen solvent. In particular, a hydrocarbon-based solvent is preferable, various solvents as components separated from petroleum are more preferable, and hydrocarbon-based solvents of aromatic groups, paraffin, naphthene, kerosine, gasoline, naphtha, heating oil, and light oil are still more preferable.

The content of the metal extractant in the oil phase is appropriately set in consideration of the content of the metal ions, the amount of coordination to the metal ions, the number of the coordinating functional groups, and the like. For example, the content in the oil phase can be 20 to 10,000 millimole/L (mM), and is preferably 50 to 1,000 millimole/L and more preferably 100 to 500 millimole/L.

The temperature of the oil phase is not particularly limited and can be, for example, 10°C to 60°C.

The oil phase may include appropriate components in addition to the metal extractant according to the embodiment of the present invention.

The oil phase can be prepared by dissolving the metal extractant in the organic solvent. Preparation conditions of the oil phase are not particularly limited. For example, the preparation temperature can be 10°C to 60°C.

### (Contact and Mixing)

In the separation recovery method according to the embodiment of the present invention, the water phase and the oil phase described above are mixed and left to stand.

In this case, mixing conditions and standing conditions are not particularly limited and can be appropriately set. For example, mixing can be performed using various mixing devices. Examples of a method using the mixing device include a method using a magnetic stirrer (stirrer tip), a method using a mechanical stirrer, and a method using a mixer. Stirring conditions (a stirring rate, a stirring time, and the like) only need to be conditions (conditions where the metal extractant is coordinate-bonded to the metal ions) where the water phase and the oil phase can be mixed, and are appropriately set depending on the combination of the metal ions and the metal extractant, and the mixing temperature, and the mixing device. For example, the stirring time is not uniquely determined depending on the stirring conditions and the like, and can be, for example, 10 minutes to 24 hours.

The standing conditions only need to be conditions where the water phase and the oil phase are separated into two layers. For example, the standing time can be 10 minutes to 24 hours after stopping mixing.

The mixing temperature and the standing temperature are not particularly limited and can be, for example, 10°C to 60°C.

During the mixing of the water phase and the oil phase, a mixing ratio between the water phase and the oil phase is appropriately set depending on a metal ion concentration, a concentration of the metal ions, the content (concentration) of the metal extractant, and the like, and is not uniquely determined. For example, in a case where the water phase and the oil phase that satisfy the respective concentrations are mixed, the ratio of the oil phase to 100 mL of the water phase can be 50 to 2,000 mL and is preferably 80 to 1,000 mL and more preferably 80 to 200 mL. On the other hand, focusing on the metal ions present in the water phase, it is preferable that the oil phase is mixed at a ratio of 0.1 to 20 moles of the metal extractant to the total content (moles) of the metal ions. In addition, the content of the metal extractant with respect to the total content of the metal ions to which the metal extractant can be coordinated (also referred to as the mixing amount; a ratio of the number of moles of the metal extractant to the total number of moles of the metal ions: molar ratio) can be, for example, 0.1 to 20.0 equivalents. Here, the metal ions to which the metal extractant can be coordinated refers to metal ions that are coordinated to the metal extractant and are extracted to the oil phase.

During the mixing of the water phase and the oil phase, the pH of the mixing system can be adjusted. Here, the pH that is set for specific metal ions to be extracted is not uniquely determined and is appropriately determined in consideration of the pKa of the metal extractant, the complex formation constants of the metal extractant and the metal ions, the number of metal ions to be coordinated, and the like. The pH of the mixing system is preferably 0.01 to 14 and can also be, for example, 2 to 14 and preferably 3 to 10. The preparation of the pH can be performed using the acid or the alkali described above, an aqueous solution thereof, or the like, and one preferable aspect is an aspect where ammonium ions are not used.

In a case where the pH of the mixing system is adjusted during the mixing of the water phase and the oil phase, the mixing of the water phase and the oil phase and the standing after the mixing are performed after adjusting the pH.

In a two-phase separated fluid (a solvent extraction phase or a solvent extraction system) where the water phase and the oil phase are phase-separated that is obtained by mixing the water phase and the oil phase and leaving the mixture to stand, the water phase and the oil phase are phase-separated into layers and present in a state where they are in contact with each other. The two or more kinds of metal ions to which the metal extractant is coordinate-bonded among the above-described plural kinds of metal ions are present (moved) in the oil phase. The two or more kinds of metal ions extracted to the oil phase among the plural kinds of metal ions are not particularly limited. For example, two or more kinds of metal ions belonging to Groups 4, 7, 9, and 10 can be used, examples thereof include a combination of Group 9 and Group 10 (excluding a combination including Group 7), a combination of Group 4 and Group 9, a combination of Group 7, Group 9, and Group 10, and a combination of Group 7, Group 8, Group 9, and Group 10. Specific examples of the combination of the valuable metal elements include a combination of Co and Ni, a combination of Zr and Rh, a combination of Mn and Co, a combination of Mn, Co, and Ni, a combination including Mn, Fe, Co, and Ni.

In addition, the number of kinds of the metal ions extracted to the oil phase is not particularly limited as long as it is 2 or more. For example, the number of kinds of the metal ions can be 2 to 10 and is preferably 2 to 6 and more preferably 2 or 3.

**In** the separation recovery method according to the embodiment of the present invention, using a simple method of mixing the water phase and the oil phase with each other and leaving the mixture to stand, specific two or more kinds of metal ions among plural kinds of metal ions can be separated, recovered, and extracted with high selectivity and high recovery rate. **In** particular, while extracting ions of two or more kinds of metal elements, one kind of metal ions can be separated and recovered to an oil phase with high selectivity and high recovery rate.

The one kind of metal ions that can be separated and recovered with high selectivity and high recovery rate are not uniquely determined depending on the group or the period of the metal ions, the content thereof, the kind of the metal extractant, and the like. For example, in a case where metal ions belonging to Group 9 and metal ions belonging to Group 10 are extracted to the oil phase, the metal ions belonging to Group 9 can be separated and recovered with high selectivity and high recovery rate. In particular, in a case where Co ions as metal ions belonging to Group 9 and Ni ions as metal ions belonging to Group 20 are extracted, the Co ions can be separated and recovered with high selectivity and high recovery rate. In addition, in a case where metal ions belonging to Group 7 and metal ions belonging to Group 9 are extracted to the oil phase, the metal ions belonging to Group 7 can be separated and recovered with high selectivity and high recovery rate. Further, in a case where metal ions belonging to Group 4 and metal ions belonging to Group 9 are extracted to the oil phase, the metal ions belonging to Group 4 can be separated and recovered with high selectivity and high recovery rate.

In the separation recovery method according to the embodiment of the present invention, as described above, two or more kinds of metal ions among plural kinds of metal ions present in the water phase can be extracted and recovered to the oil phase with high selectivity and high recovery rate. In particular, in the separation recovery method according to the embodiment of the present invention, while extracting ions of two or more kinds of metal elements, one kind of metal ions can be separated and recovered to the oil phase with high selectivity and high recovery rate. Therefore, by further subjecting the water phase including the two or more kinds of metal ions stripped from the oil phase to the separation recovery method according to the embodiment of the present invention, the selectivity of one kind of metal ions can be further improved without significant deterioration in recovery rate, and thus high-purity metal ions can be recovered with high recovery rate.

The separation recovery method according to the embodiment of the present invention can also be a method of extracting two or more kinds of metal ions.

The separation recovery method according to the embodiment of the present invention may include steps other than the step of mixing the water phase and the oil phase with each other and leaving the mixture to stand. Examples of the other steps include a method of stripping (isolating) metal ions from the oil phase obtained in the step of mixing the water phase and the oil phase with each other and leaving the mixture to stand, a step of recovering the stripped metal ions as a compound (salt), a step of purifying the stripped metal ions or the compound thereof, and a step of removing ions of metal elements belonging to Group 1 or Group 2 in the periodic table of elements in advance. As a method of stripping (isolating) the metal ions from the oil phase, a well-known method can be applied without any particular limitation. For example, the different-group metal ions can be stripped by adjusting the liquid phase to be acidic, for example, pH of 2 to 4 using an inorganic acid such as sulfuric acid, hydrochloric acid, or nitric acid. As a method of recovering the stripped metal ions as a compound, a well-known method can be applied without any particular limitation.

### <Separation Recovery Method I according to Embodiment of Present Invention>

A separation recovery method I according to the embodiment of the present invention is a method of using the above-described metal extractant I according to the embodiment of the present invention as a metal extractant in the separation recovery method according to the embodiment of the present invention.

The separation recovery method I according to the embodiment of the present invention can be performed using the same method as that of the separation recovery method I according to the embodiment of the present invention. In the separation recovery method I according to the embodiment of the present invention, in the above-described range, the pH of the water phase is more preferably 0.5 to 8.0 from the viewpoints of the selectivity and the recovery rate and is still more preferably 3.0 to 8.0 and still more preferably 5.0 to 7.0 from the viewpoint that the recovery rate can be particularly improved. In the separation recovery method I according to the embodiment of the present invention, the pH of the mixing system of the water phase and the oil phase is preferably set in the above-described range. The pH of the mixing system is more preferably 0.5 to 9.0 from the viewpoints of the selectivity and the recovery rate and is still more preferably 2.5 to 8.0 and still more preferably 4.0 to 7.0 from the viewpoint that the recovery rate can be particularly improved.

In the separation recovery method I according to the embodiment of the present invention, the metal extractant I alone can be coordinated to metal ions to extract the metal ion to the oil phase. Therefore, the water phase and the oil phase do not need to include a compound that acts to extract metal ions in cooperation with the metal extractant **I, for** example, a compound that is coordinated to metal ions such as a well-known metal extractant. **In** the separation recovery method I according to the embodiment of the present invention, typically, the water phase including a plurality of metal ions as an essential component and the oil phase including the metal extractant I as an essential component are used.

**In** the separation recovery method I according to the embodiment of the present invention, as in the separation recovery method according to the embodiment of the present invention, two or more kinds of metal ions among plural kinds of metal ions present in the water phase can be extracted and recovered to the oil phase with high selectivity and high recovery rate.

### <Separation Recovery Method II according to Embodiment of Present Invention>

A separation recovery method II according to the embodiment of the present invention is a method of using the above-described metal extractant II according to the embodiment of the present invention as a metal extractant in the separation recovery method according to the embodiment of the present invention.

The separation recovery method II according to the embodiment of the present invention can be performed using the same method as that of the separation recovery method according to the embodiment of the present invention, and two or more kinds of metal ions among plural kinds of metal ions present in the water phase can be extracted and recovered to the oil phase with high selectivity and high recovery rate.

Different points of the separation recovery method II according to the embodiment of the present invention from the separation recovery method according to the embodiment of the present invention will be described.

Since the metal extractant II is an amine-based (basic) extractant, metal ions extracted to the oil phase need to be negatively charged. Therefore, in the separation recovery method II according to the embodiment of the present invention, the water phase includes a compound for forming a coordinating complex (complex ions) that is coordinated to metal ions to be negatively charged, and the metal ions form negatively charged complex ions.

Examples of this compound include a compound that is coordinated to metal ions to form negatively charged complex ions and a compound from which anions are generated and coordinated to metal ions to form negatively charged complex ions. The compound that is coordinated to metal ions is not particularly limited, and examples thereof include a chelating agent such as sodium nitrilotriacetate. In addition, examples of the compound from which anions are generated and coordinated to metal ions include a metal salt of an inorganic acid or an organic acid such as a nitrate, a sulfate, or a hydrochloride and a halide. Examples of the halide include an inorganic halide and an organic halide. Examples of the inorganic halide include halides of typical elements (elements belonging to Group 1, 2, and 12 to 18 in the periodic table), specifically, lithium chloride, sodium chloride, or potassium chloride.

The compound that forms complex ions is preferably the compound from which anions are generated and coordinated to metal ion, and is more preferably an inorganic halide.

The water phase including the compound that forms a negatively charged coordinating complex (complex ions) preferably includes anions generated (dissociated) from the compound and more preferably includes halide ions. In the present invention, the water phase including anions or halide ions represents that not only anions or halide ions dissolved in the water phase and present (free) alone but also anions or halide ions coordinated to metal ions are included.

The concentration of the compound in the water phase is not particularly limited as long as complex ions can be formed. For example, the concentration can be 1 to 1000 g/L and preferably 10 to 300 g/L.

The content (including the compound coordinated to metal ions; that has the same definition as the addition amount to the water phase) of the compound that forms negatively charged metal ions in the water phase is not particularly limited as long as the compound can be coordinated to metal ions to form negatively charged complex ions, and is appropriately set in consideration of the content of the metal ions, the amount of the compound coordinated to the metal ions, the number of coordinating functional groups, and the like. The content of the compound with respect to 100 parts by mass of the total content of the metal ions (including metal ions that are not coordinated) in the water phase can be 10 to 10000 parts by mass and is preferably 100 to 1000 parts by mass. In addition, the content of the compound with respect to the total content of the metal ions (including metal ions that are not coordinated) in the water phase can be 0.1 to 100 moles and is preferably 1.0 to 100 moles. On the other hand, the content (mixing amount: molar ratio) of the compound with respect to the total content of the metal ions can be, for example, 0.1 to 100 equivalents and is preferably 1.0 to 50 equivalents.

The water phase can be prepared by dissolving metal ions and the compound in water, and can also be prepared by mixing an aqueous solution in which the metal ions are dissolved and an aqueous solution in which the compound is dissolved with each other. In order to coordinate-bond the compound to at least one kind of metal ions, it is preferable to prepare the water phase at a preparation temperature of 10°C to 60°C and pH of 0.1 to 10 for 10 minutes to 6 hours. In order to adjust the pH, the acid or the alkali described above can be used.

The separation recovery method II according to the embodiment of the present invention can be performed using the same method as that of the separation recovery method II according to the embodiment of the present invention, except that complex ions of metal ions are formed in the water phase. In the separation recovery method II according to the embodiment of the present invention, in the above-described range, the pH of the water phase is more preferably 0.5 to 7 from the viewpoints of the selectivity and the recovery rate and is still more preferably 3.0 to 7.0 from the viewpoint that the recovery rate can be particularly improved. In the separation recovery method II according to the embodiment of the present invention, the pH of the mixing system of the water phase and the oil phase is preferably set in the above-described range. The pH of the mixing system is more preferably 0.01 to 5.5 from the viewpoints of the selectivity and the recovery rate and is still more preferably 0.1 to 4.5 and still more preferably 0.1 to 3.5 from the viewpoint that the recovery rate can be particularly improved.

In the separation recovery method II according to the embodiment of the present invention, the metal extractant II can be coordinated to metal ions forming complex ions to extract the metal ion to the oil phase. Therefore, the water phase and the oil phase do not need to include a compound, such as a well-known metal extractant, that relates to extraction of metal ions other than the compound that is coordinated to metal ions to form complex ions and the metal extractant II.

In the separation recovery method II according to the embodiment of the present invention, the water phase including a plurality of metal ions as an essential component and the compound that forms complex ions of the metal ions and the oil phase including the metal extractant II as an essential component are used.

In the separation recovery method II according to the embodiment of the present invention, as in the separation recovery method according to the embodiment of the present invention, two or more kinds of metal ions among plural kinds of metal ions present in the water phase can be extracted and recovered to the oil phase with high selectivity and high recovery rate.

### Examples

Hereinafter, the present invention will be described in more detail based on Examples but is not limited to these examples. "Parts" and "%" that represent compositions in the following examples are mass-based unless particularly otherwise described.

### [Synthesis and Preparation of Metal Extractant]

Metal extractants shown below were synthesized and prepared.

In the following metal extractants E-6 and T-5, "Me" represents a methyl group. 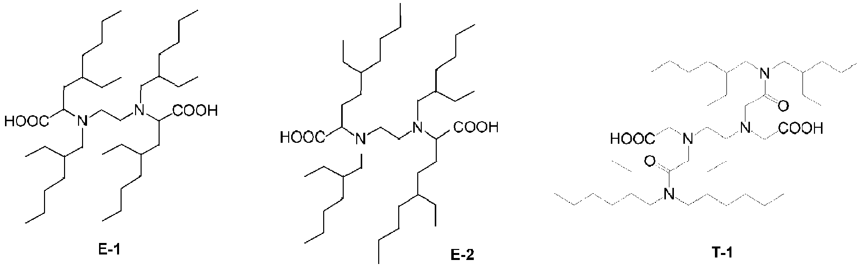 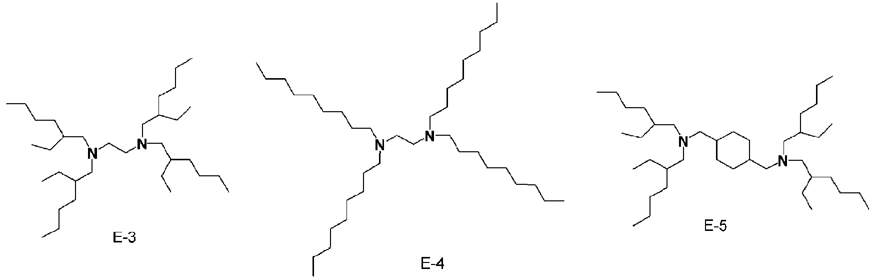 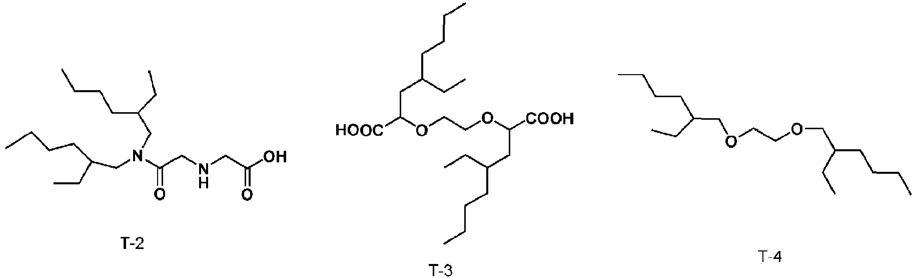 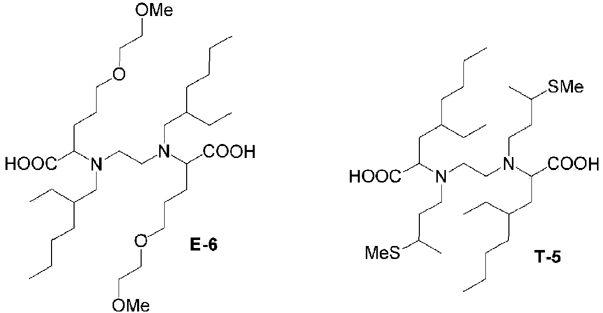
PC-88A: mono-2-ethylhexyl (2-Ethylhexyl)phosphonate shown below (manufactured by Tokyo Chemical Industry Co., Ltd.) 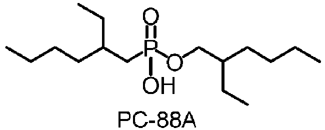
VA-10: Versatic acid 10 (manufactured by Hexion Specialty Chemicals, Inc.)
   EDTA: ethylenediaminetetraacetic acid, manufactured by DOJINDO LABORATORIES
   Tris-2-EHA: tris(2-ethylhexyl)amine shown below (manufactured by BASF SE)

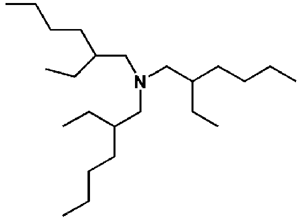

### <Synthesis of Metal Extractant E-1>

A metal extractant E-1 was synthesized as follows.

That is, 305 g of ethanol and 46.0 g of sodium ethoxide (manufactured by FUJIFILM Wako Pure Chemical Corporation) were added to a 1 L three-necked eggplant flask and were stirred well. 97.5 g of diethyl malonate, 112 g of 1-bromo-2-ethylhexane, and 126 g of ethanol were added and were stirred in a reflux state for 6 hours. The obtained solution was filtered through a Buchner funnel, the filtrate was concentrated, and 200 mL of 2 M hydrochloric acid was added. The obtained solution was extracted with 500 mL of ethyl acetate and subsequently was dried with sodium sulfate. After removing sodium sulfate, the solvent was concentrated.

The obtained compound was added to a 2L three-necked eggplant flask, 550 g of ethanol and 715 g of a 4 M (mole/L) NaOH aqueous solution were added, and the solution was stirred in a reflux state for 7 hours. The solution was allowed to cool at room temperature and was extracted with xylene to obtain a xylene solution.

The obtained xylene solution was added to 1 L three-necked eggplant flask and was stirred in a reflux state for 8 hours. After allowing the solution to cool at room temperature, an 1M NaOH aqueous solution and water were added and extracted with toluene, and the solvent was distilled off under a reduced pressure to obtain a compound A.

35.0 g of the obtained compound A and 121 g of thionyl chloride were added to a 500 mL three-neck flask and were stirred at an internal temperature of 55°C for 2 hours. 97.4 g of bromine was added, and the solution was further stirred at an internal temperature of 55°C for 5 hours. After distilling off thionyl chloride and bromine under a reduced pressure, iced water was added, the solution was extracted with toluene, and the solvent was distilled off under a reduced pressure to obtain a compound B.

5.0 g of the following intermediate I-1, 33.3 g of ethanol, 16.7 g of water, and 8.3 g of NaOH were added to a 300 mL three-necked eggplant flask and were stirred in a reflux state. A solution in which 13.3 g of the compound B was dissolved in 25.0 g of ethanol was added dropwise for 1 hour and was stirred as it is for 36 hours. After leaving the solution to stand at room temperature, hydrochloric acid was added to neutralize the solution to pH to 3 to 4. The obtained solution was extracted with toluene, and the solvent was distilled off under a reduced pressure to obtain 6.5 g (yield: 59%) of a metal extractant E-1 as a yellow liquid.

### <Synthesis of Intermediate I-1>

20.0 g of ethylenediamine, 350 g of ethanol, and 87.5 g of 2-ethylhexanal were added to 1 L three-necked eggplant flask and were stirred at room temperature for 5 hours. While cooling the reaction solution in an ice bath, 37.8 g of sodium borohydride was added, the ice bath was removed, and the solution was stirred at room temperature for 1 hour. While cooling the reaction solution in an ice bath, an ammonium chloride solution was added until the solution did not foam, and ethanol was distilled off under a reduced pressure. By extracting the obtained solution with toluene and distilling off the solvent under a reduced pressure, the intermediate I-1 (N,N'-bis(2-ethylhexyl)ethane1,2-diamine) was obtained with a yield of 98%.

The metal extractant E-1 synthesized as described above was identified as follows.

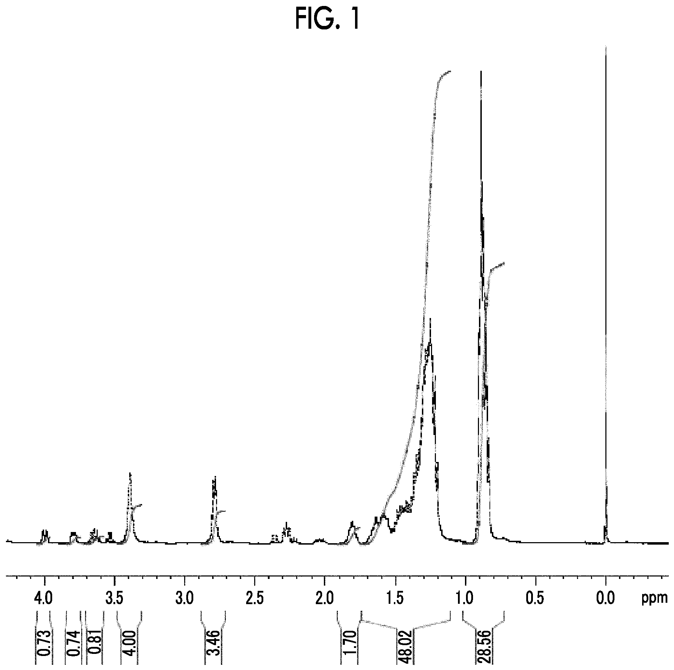

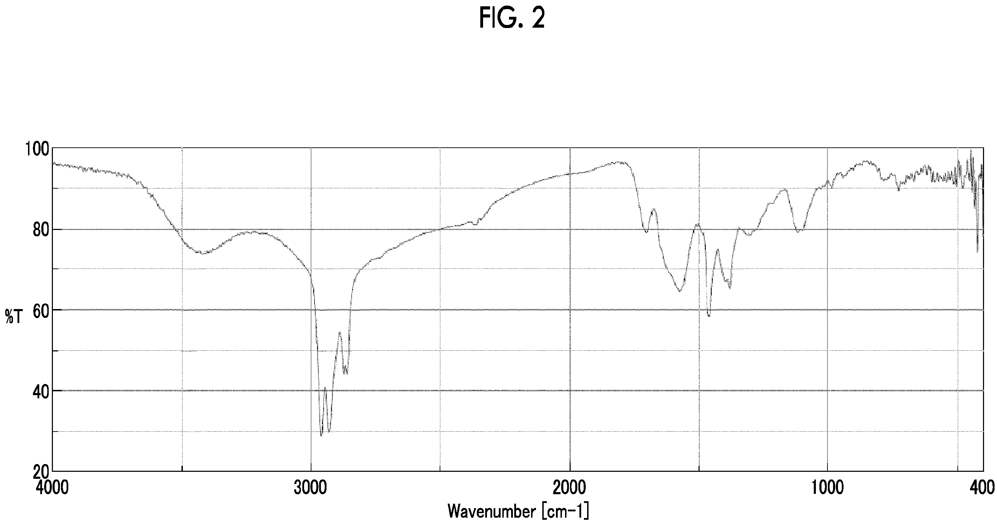

That is, E-1 was dissolved in deuterated chloroform, and ¹H-NMR was measured (device: BLUKER 400). The obtained chart is shown in Fig. 1. In addition, the metal extractant E-1 and KBr were mixed, and FT-IR measurement was performed. The obtained chart is shown in Fig. 2.

In the ¹H-NMR chart shown in Fig. 1, a peak derived from a diastereomer at a chemical shift δ of 3.5 to 4.0 ppm was verified. Accordingly, it is estimated that at least two asymmetric centers derived from 2-ethylhexyl were introduced into the compound. In addition, it can seen that, with respect to an integrated value 4H of a peak (δ3.39) derived from methylene positioned between two N atoms of ethylenediamine, a peak (δ0.89) derived from a terminal methyl group of the 2-ethylhexyl group corresponds to 24H. Accordingly, it can be seen that four 2-ethylhexyl groups were introduced.

In addition, in the FT-IR chart shown in Fig. 2, OH stretching derived from a carboxyl group is estimated from a broad peak shown in the vicinity of 3400 cm⁻¹.

As a result, the obtained compound was identified to have the above-described structure represented by E-1.

### <Synthesis of Metal Extractant E-2>

A metal extractant E-2 was synthesized using the same method as that of the synthesis of the metal extractant E-1, except that 1-bromo-2-ethylhexane was changed to 1-bromo-3-ethylheptane. The obtained metal extractant E-2 was identified using the same method as that of the metal extractant E-1.

### <Synthesis of Metal Extractant E-3>

A metal extractant E-3 was synthesized as follows.

That is, 25.0 g of 1,2-dibromoethane and 129 g of di(2-ethylhexyl)amine were added to a 300 mL three-necked eggplant flask and stirred at an internal temperature of 100°C for 10 hours. An excess amount of di(2-ethylhexyl)amine was distilled off under a reduced pressure from the obtained solution to obtain a metal extractant E-3 with a yield of 85%.

The metal extractant E-3 synthesized as described above was identified as follows.

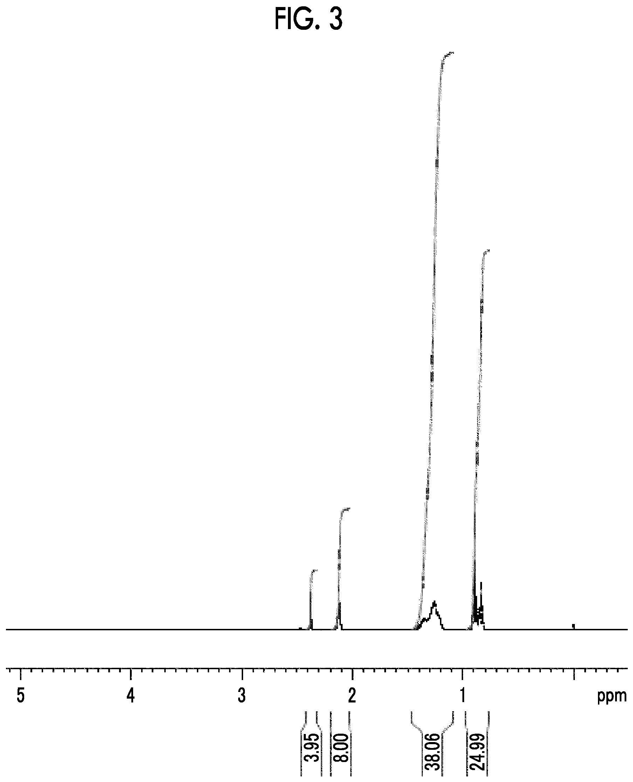

That is, the metal extractant E-3 was dissolved in deuterated chloroform, and ¹H-NMR was measured (device: BLUKER 400). The obtained chart is shown in Fig. 3. In addition, fragment-ions of the compound were detected by GC-MS (device: GCMS-QP2010).

In addition, it can seen that, in the ¹H-NMR chart shown in Fig. 1, with respect to an integrated value 4H of a peak (δ2.46) derived from methylene positioned between two N atoms of ethylenediamine, peaks (δ2.11, δ1.26, and δ0.89) derived from the 2-ethylhexyl group correspond to 8H, 36H, and 24H, respectively. Accordingly, it can be seen that four 2-ethylhexyl groups were introduced. In addition, the following structure was estimated from fragmentation of m/z 254 obtained by GC-MS.

As a result, the obtained compound was identified to have the above-described structure represented by E-3.

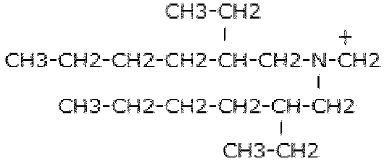

### <Synthesis of Metal Extractant E-4>

A metal extractant E-4 was synthesized using the same method as that of the synthesis of the metal extractant E-3, except that di(2-ethylhexyl)amine was changed to dinonylamine. The obtained metal extractant E-4 was identified using the same method as that of the metal extractant E-3.

### <Synthesis of Metal Extractant E-5>

A metal extractant E-5 was synthesized using the same method as that of the synthesis of the metal extractant E-3, except that 1,2-dibromoethane was changed to 1,4-bis(bromomethyl)cyclohexane. The obtained metal extractant E-5 was identified using the same method as that of the metal extractant E-3.

### <Synthesis of Metal Extractant E-6>

A metal extractant E-6 was synthesized using the same method as that of the synthesis of the metal extractant E-1, except that 1-bromo-2-ethylhexane was changed to 1-bromo-3-(2-methoxyethoxy)propane. The obtained metal extractant E-6 was identified using the same method as that of the metal extractant -1.

### <Synthesis of Metal Extractant T-1>

A metal extractant T-1 was synthesized and identified using a method described in JP2016-028021A.

### <Synthesis of Metal Extractant T-2>

A metal extractant T-2 was synthesized and identified using a synthesis method of D2EHAG described in JP2014-205900A.

### <Synthesis of Metal Extractant T-3>

A metal extractant T-3 was synthesized using the same method as that of the synthesis of the metal extractant E-1, except that the intermediate I-1 was changed to ethylene glycol. The obtained metal extractant T-3 was identified using the same method as that of the metal extractant E-1.

### <Synthesis of Metal Extractant T-4>

A metal extractant T-4 was synthesized using the same method as that of the synthesis of the metal extractant E-3, except that di(2-ethylhexyl)amine was changed to 2-ethylhexanol and 60.0 g of sodium hydroxide was added. The obtained metal extractant T-4 was identified using the same method as that of the metal extractant E-3.

### <Synthesis of Metal Extractant T-5>

A metal extractant T-5 was synthesized using the same method as that of the synthesis of the metal extractant E-1, except that 2-ethylhexanal was changed to 3-(methylthio)butanal during the synthesis of the intermediate I-1. The obtained metal extractant T-5 was identified using the same method as that of the metal extractant -1.

Regarding the synthesized or prepared metal extractant, the molecular weight and the pKa calculated using the above-described method are shown in Table 1-2.

### [Preparation of Metal Ion-Containing Aqueous Solution]

### <Preparation of Metal Ion-Containing Aqueous Solution (Water Phase) used in Separation Recovery Method I using Metal Extractant I>

95.4 g of cobalt (II) sulfate heptahydrate (manufactured by FUJIFILM Wako Pure Chemicals Corporation) and 95.7 g of nickel (II) sulfate heptahydrate (manufactured by FUJIFIILM Wako Pure Chemicals Corporation) were added to a 1L measuring flask, were diluted with ultrapure water, and were stirred and dissolved at 30°C. As a result, a metal ion-containing aqueous solution (W1) including two kinds of metal ions was prepared.

In addition, each of sulfates of a combination of metal ions shown in Table 1-1 was dissolved in ultrapure water to prepare each of metal ion-containing aqueous solutions (W2) and (W3) including two or three kinds of metal ions.

### <Preparation of Metal Ion-Containing Aqueous Solution (Water Phase) used in Separation Recovery Method II using Metal Extractant II>

200 g of lithium chloride (manufactured by FUJIFILM Wako Pure Chemical Corporation), 95.4 g of cobalt (II) sulfate heptahydrate (manufactured by FUJIFILM Wako Pure Chemicals Corporation), and 95.7 g of nickel (II) sulfate heptahydrate (manufactured by FUJIFILM Wako Pure Chemicals Corporation) were added to a 1L measuring flask, were diluted with ultrapure water, and were stirred and dissolved at 30°C. As a result, a metal ion-containing aqueous solution (W4) including two kinds of metal ions was prepared. In this aqueous solution, in the two kinds of metal ions (cobalt and nickel), complex ions to which halide ions were coordinated were formed.

### <Preparation of Metal Extractant Solution (Oil Phase)>

Each of the synthesized or prepared metal extractants was added to a 100 mL measuring flask, and was diluted using kerosine (manufactured by FUJIFILM Wako Pure Chemicals Corporation) at room temperature. As a result, metal extractant solutions (Y1) to (Y6) and (Yc1) to (Yc9) (concentration: 310 mM) including the metal extractants were prepared, respectively.

A metal extractant EDTA was not able to be dissolved in kerosine, and a metal extractant solution (Yc4) was not able to be prepared.

### [Examples 1 to 4 and 8: Separation Recovery Method I using Metal Extractant I and Comparative Examples 1 to 6 and 9]

### <Example 1>

12 mL of the extractant (Y1) with respect to 10 mL of the prepared metal ion-containing aqueous solution (W1) was added to a 30 mL vial tube, and the mixture was stirred using a stirrer tip at 25°C for 30 minutes. In this case, the mixing amount (unit: equivalent) of the metal extractant with respect to the total content of metal ions to be coordinated (that have the same definition as the extracted metal ions; in Example 1, Co and Ni) was 0.5. Next, a 10 M sodium hydroxide aqueous solution or a 10 M hydrochloric acid was added to adjust the pH of the mixed solution to a value shown in the column "pH during Mixing" of Table 1-2. Further, the solution was stirred at 25°C for 30 minutes, and was left to stand at the same temperature for 1 hour. After verifying that the solution was separated into two layers of the organic phase (oil phase) and the water phase, the separated water phase was extracted, and the separation and recovery of the metal ions was performed. The metal ions extracted in Example 1 and the metal ions extracted in the maximum amount are shown in the column "Kind" and the column "Maximum Amount Extracted" of the column "Extracted Metal Ions" in Table 1-2, respectively.

### <Examples 2 to 4 and 8 and Comparative Examples 1 to 6 and 9>

Separation and recovery of metal ions according to Examples 2 to 4 and 8 and Comparative Examples 1 to 6 and 9 were performed using the same method as that of Example 1, except that the metal ion-containing aqueous solution and the metal extractant solution were changed to a combination shown in Table 1-1 and Table 1-2 (hereinafter, both of which are referred to as Table 1"), the pH during the mixing of the water phase and the oil phase was set to a value shown in the column "pH during Mixing" of Table 1-2, and the mixing amount (unit: equivalent) of the metal extractant with respect to the total content of metal ions to be coordinated was set to a value shown in the column "Mixing Amount" of Table 1-2 for mixing and standing. The metal ions extracted in each of Examples and the metal ions extracted in the maximum amount are shown in the column "Kind" and the column "Maximum Amount Extracted" of the column "Extracted Metal Ions" in Table 1-2, respectively.

In Comparative Example 4 using the metal extractant solution (Yc4), metal ions were not able to be extracted in the oil phase. Therefore, the column "Extracted Metal Ions", the column "Selection Ratio", and the like in Table 1-2 are shown as "-".

### [Examples 5 to 7: Separation Recovery Method II using Metal Extractant II, and Comparative Examples 7 and 8]

### <Example 5>

12 mL of the extractant (Y3) with respect to 10 mL of the prepared metal ion-containing aqueous solution (W4) was added to a 30 mL vial tube, and the mixture was stirred using a stirrer tip at 25°C for 30 minutes. In this case, the mixing amount (unit: equivalent) of the metal extractant to the total content of the metal ions was 0.5. Next, a 10 M sodium hydroxide aqueous solution or a 10 M hydrochloric acid was added to adjust the pH of the mixed solution to a value shown in the column "pH during Mixing" of Table 1-2. Further, the solution was stirred at 25°C for 30 minutes, and was left to stand at the same temperature for 1 hour. After verifying that the solution was separated into two layers of the organic phase (oil phase) and the water phase, the separated water phase was extracted, and the separation and recovery of the metal ions was performed. The metal ions extracted in Example 5 and the metal ions extracted in the maximum amount are shown in the column "Kind" and the column "Maximum Amount Extracted" of the column "Extracted Metal Ions" in Table 1-2, respectively.

### <Examples 6 and 7 and Comparative Examples 7 and 8>

The separation and recovery of the metal ions according to Examples 6 and 7 and Comparative Examples 7 and 8 was performed using the same method as that of Example 5, except that the metal ion-containing aqueous solution and the extractant solution were changed to a combination shown in Table 1-2, and the pH during the mixing of the water phase and the oil phase was set to a value shown in the column "pH during Mixing" of Table 1 for mixing and standing. The metal ions extracted in each of Examples and the metal ions extracted in the maximum amount are shown in the column "Kind" and the column "Maximum Amount Extracted" of the column "Extracted Metal Ions" in Table 1-2, respectively.

Regarding each of the water phases used in Examples and Comparative Examples and the water phases after the extraction, the pH was measured using a pH meter (SK-620 pH II, manufactured by SATOTECH), and each of the contents of dissolved metal ions was determined using an inductively coupled plasma-optical emission spectrometer (ICP-OES) (Optima 7300 D (trade name), manufactured by Perkin Elmer Co., Ltd.). Measured values of the pH of each of the water phases used in Examples and Comparative Examples and the content of dissolved metal ions in each of the water phases are shown in the column "Water Phase pH" of Table 1-2 and the column "Metal Ion Concentration (ppm)" of Table 1-1. The maximum amount (ppm) of the metal ions extracted measured as described above was divided by the total amount (ppm) of the other metal ions extracted to calculate a ratio between the amounts thereof extracted. The result is shown in the column "Selection Ratio" of Table 1-2.

In addition, in Examples and Comparative Examples, the results measured using the same method as that of the pH during the mixing of the water phase and the oil phase are shown in the column "pH during Mixing". Further, the mixing amount of the metal extractant with respect to the total content of the metal ions to be coordinated is shown in the column "Mixing Amount" of Table 1-2. In Table 1-2, the unit of the mixing amount is equivalent but is not shown.

In a case where the same experiment was performed using the same method as that of Examples 1 to 8 and Comparative Examples 1 to 9, except that the metal ion concentration in the water layer was reduced to 1/5, the same results were obtained.

**[Table 1]**

| No. | Water Phase | Metal Ion Concentration in Water Phase before Extraction (ppm) | | | | | Metal Ion Concentration in Water Phase after Extraction (ppm) | | | | |
|---|---|---|---|---|---|---|---|---|---|---|---|
| | | Zr | Mn | Co | Ni | Rh | Zr | Mn | Co | Ni | Rh |
| Example 1 | W1 | 0 | 0 | 20,000 | 20,000 | 0 | 0 | 0 | <1 | 18,000 | 0 |
| Example 2 | W2 | 0 | 20,000 | 20,000 | 20,000 | 0 | 0 | <1 | 19,000 | 20,000 | 0 |
| Example 3 | W3 | 20,000 | 0 | 0 | 0 | 20,000 | <1 | 0 | 0 | 0 | 17,000 |
| Example 4 | W1 | 0 | 0 | 20,000 | 20,000 | 0 | 0 | 0 | <1 | 15,000 | 0 |
| Comparative Example 1 | W2 | 0 | 20,000 | 20,000 | 20,000 | 0 | 0 | <1 | 10,000 | 16,000 | 0 |
| Comparative Example 2 | W2 | 0 | 20,000 | 20,000 | 20,000 | 0 | 0 | <1 | 4,000 | 6,000 | 0 |
| Comparative Example 3 | W2 | 0 | 20,000 | 20,000 | 20,000 | 0 | 0 | <1 | 1,000 | 1,500 | 0 |
| Comparative Example 4 | W2 | 0 | 20,000 | 20,000 | 20,000 | 0 | 0 | 20,000 | 20,000 | 20,000 | 0 |
| Comparative Example 5 | W2 | 0 | 20,000 | 20,000 | 20,000 | 0 | 0 | <1 | 3,000 | 5,000 | 0 |
| Comparative Example 6 | W1 | 0 | 0 | 20,000 | 20,000 | 0 | 0 | 0 | <1 | 2,000 | 0 |
| Example 5 | W4 | 0 | 0 | 20,000 | 20,000 | 0 | 0 | 0 | <1 | 16,000 | 0 |
| Example 6 | W4 | 0 | 0 | 20,000 | 20,000 | 0 | 0 | 0 | <1 | 14,000 | 0 |
| Example 7 | W4 | 0 | 0 | 20,000 | 20,000 | 0 | 0 | 0 | <1 | 15,000 | 0 |
| Comparative Example 7 | W4 | 0 | 0 | 20,000 | 20,000 | 0 | 0 | 0 | 5,000 | 10,000 | 0 |
| Comparative Example 8 | W4 | 0 | 0 | 20,000 | 20,000 | 0 | 0 | 0 | 17,000 | 18,000 | 0 |
| Example 8 | W1 | 0 | 0 | 20,000 | 20,000 | 0 | 0 | 0 | <1 | 16,000 | 0 |
| Comparative Example 9 | W1 | 0 | 0 | 20,000 | 20,000 | 0 | 0 | 0 | 13,000 | 14,000 | 0 |

**[Table 2]**

| No. | Extracted Metal Ions | | Water Phase pH | pH during Mixing | Oil Phase | Metal Extractant | | | | Selection Ratio |
|---|---|---|---|---|---|---|---|---|---|---|
| | Kind | Maximum Amount Extracted | | | | | Molecular Weight | pKa | Mixing Amount | |
| Example 1 | Co,Ni | Co | 6.8 | 6.8 | Y1 | E-1 | 625 | 6.1 | 0.5 | 10 |
| Example 2 | Mn,Co | Mn | 6.2 | 5.5 | Y1 | E-1 | 625 | 6.1 | 0.4 | 20 |
| Example 3 | Zr,Rh | Zr | 6.3 | 4.2 | Y1 | E-1 | 625 | 6.1 | 0.7 | 6.7 |
| Example 4 | Co,Ni | Co | 6.8 | 6.9 | Y2 | E-2 | 653 | 6.3 | 0.5 | 4.0 |
| Comparative Example 1 | Mn,Co,Ni | Mn | 6.2 | 4.5 | Yel | PC-88A | 306 | 4.6 | 0.4 | 1.4 |
| Comparative Example 2 | Mn,Co,Ni | Mn | 6.2 | 6.8 | Yc2 | VA-10 | 172 | 7.9 | 0.4 | 0.7 |
| Comparative Example 3 | Mn,Co,Ni | Mn | 6.2 | 2.4 | Yc3 | T-1 | 739 | 6.0 | 0.4 | 0.5 |
| Comparative Example 4 | - | - | - | - | Yc4 | EDTA | 292 | 0.9 | 0.4 | - |
| Comparative Example 5 | Mn,Co,Ni | Mn | 6.2 | 5.5 | Yc5 | T-2 | 357 | 5.2 | 0.4 | 0.6 |
| Comparative Example 6 | Co,Ni | Co | 6.8 | 6.5 | Yc6 | T-3 | 403 | 5.8 | 0.5 | 1.1 |
| Example 5 | Co,Ni | Co | 6.2 | 1.5 | Y3 | E-3 | 509 | 11.1 | 0.5 | 5.0 |
| Example 6 | Co,Ni | Co | 6.2 | 1.5 | Y4 | E-4 | 565 | 11.4 | 0.5 | 3.3 |
| Example 7 | Co,Ni | Co | 6.2 | 1.5 | Y5 | E-5 | 591 | 11.5 | 0.5 | 4.0 |
| Comparative Example 7 | Co,Ni | Co | 6.2 | 1.5 | Yc7 | Tris-2-EHA | 354 | 5.2 | 0.5 | 1.5 |
| Comparative Example 8 | Co,Ni | Co | 6.2 | 2.0 | Yc8 | T-4 | 287 | 13.4 | 0.5 | 1.5 |
| Example 8 | Co,Ni | Co | 6.8 | 6.8 | Y6 | E-6 | 634 | 6.2 | 0.5 | 5.0 |
| Comparative Example 9 | Co,Ni | Co | 6.8 | 5.6 | Yc9 | T-5 | 605 | 6.1 | 0.5 | 1.2 |

The following can be seen from a comparison result between "Metal Ion Concentration in Water Phase before Extraction (ppm)" and "Metal Ion Concentration in Water Phase after Extraction (ppm)" shown in Table 1.

In both of Comparative Examples 1 to 3 using PC-88A, VA-10, and T-1 as acidic metal extractants in the related art in the separation recovery of metal ions from the water phase, the three kinds of metal ions present in the metal ion-containing aqueous solution (W2) can be extracted to the oil phase. However, although the recovery amount of Mn ions was high, relatively large amounts of Co and Ni ions were also extracted. Therefore, the selectivity of the Mn ions extracted in the maximum amount was low as shown in the column "Selection Ratio" of Table 1-2. In Comparative Example 4 where EDTA was used as the metal extractant, as described above, even metal ions were not able to be extracted. In addition, likewise, in all of the results of Comparative Examples 5 to 9 where the metal extractants T-2 to T-4, T-5, and Tris-2-EHA were used, the selectivity of the metal ions extracted in the maximum amount was low. In particular, in Comparative Example 8, the amount of the metal ions extracted in the maximum amount was also not sufficient.

On the other hand, in Examples 1 to 8 where the metal extractant I or II according to the embodiment of the present invention was used, two or kinds of metal ions among the metal ions present in the metal ion-containing aqueous solution were able to be extracted to the oil phase. In addition, the metal ions extracted in the maximum amount (in Examples 1 to 4 to 8: Co ions, Example 2: Mn ions, Example 3: Zr ions) can be extracted from the water phase to the oil phase substantially in the entire amount with high selectivity with respect to the metal ions other than the metal ions extracted in the maximum amount. Particularly in Examples 1 to 4 to 8, while extracting valuable metal elements, for example, both of Co ions and Ni ions important for manufacturing a lithium ion battery, one kind of metal ions: Co ions among the same-period different-group metal ions having similar physical behaviors and similar chemical behaviors can be recovered with high selectivity and high recovery rate. This way, it is considered that the acidic metal extractant according to the embodiment of the present invention has the specific function of being selectively coordinated to specific metal ions, desirably, two or more kinds of metal ions belonging to different groups among plural kinds of metal ions present in the water phase, in particular, two or more kinds of metal ions belonging to different groups. As a result, it was finally found that one kind of metal ions among the plural kinds of metal ions, in particular, the metal ions belonging to Groups 9 and 10 that can be recovered from waste LiB or the like can be separated and recovered with high selectivity and high recovery rate.

From the above results, it can be seen that, by stripping the oil phase obtained in each of Examples using a typical method and conditions, the metal ions can be separated and recovered to the oil phase simply with high selectivity and high recovery rate in a large recover amount without deterioration in high selectivity.

Incidentally, in the technique of recovering specific metal ions from the water phase including a plurality of metal ions, it is generally difficult to recover the specific metal ions with high selectivity and recovery rate, and in a case where high selectivity is maintained, the recovery rate decreases. Therefore, in order to achieve a predetermined recovery rate, currently, it is required to perform the separation recovery operation multiple times. On the other hand, according to the present invention, using the simple method, one kind of metal ions among two kinds of different-group metal ions can be extracted from the water phase to the oil phase substantially in the entire amount with high selectivity. Therefore, in consideration of the current conditions, the technical significance of the present invention is high from the viewpoint that, through the stripping step or the like from the obtained oil phase, one kind of metal ions can be recovered simply and with a small number of steps while further improving selectivity with high recovery rate.

The present invention has been described using the embodiments. However, unless specified otherwise, any of the details of the above description is not intended to limit the present invention and can be construed in a broad sense within a range not departing from the concept and scope of the present invention disclosed in the accompanying claims.

The present application claims priority based on JP2022-147027 filed on September 15, 2022, the entire content of which is incorporated herein by reference.

## Claims

1. A metal extractant for extracting metal ions present in a water phase to an oil phase,
wherein nitrogen atoms positioned at both terminals of a molecular chain forming the metal extractant do not form a carbamoyl bond and include a group including an unsubstituted hydrocarbon group and any coordinating functional group in a group G1 of coordinating functional groups below as a coordinating functional group (α) for metal ions to be extracted, or include an unsubstituted hydrocarbon group,
<group G1 of coordinating functional groups>
a hydroxyl group, an ether group, a thiol group, a thioether group, a nitrile group, an isonitrile group, a carboxy group, an amino group, an amide group, a phosphate group, a phosphonate group, a sulfonate group, a sulfinate group, a phosphino group, a nitrogen-containing heterocycle, an oxygen-containing heterocycle, and a sulfur-containing heterocycle.

2. The metal extractant according to claim 1,
wherein the group including the coordinating functional group is a hydrocarbon group including a coordinating functional group.

3. The metal extractant according to claim 1,
wherein at least one of the coordinating functional groups is selected from a carboxy group, a phosphate group, a phosphonate group, a sulfonate group, or a sulfinate group.

4. The metal extractant according to claim 1, which is used for extracting and separating metal ions of two or more elements belonging to different groups in a periodic table among the metal ions.

5. The metal extractant according to claim 1,
wherein the metal extractant is represented by Formula (I), 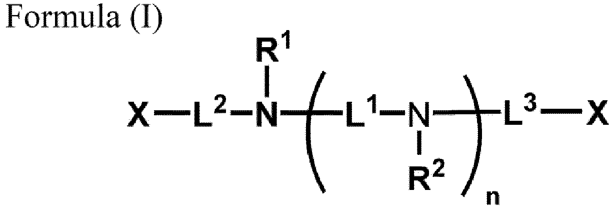
in Formula (I), R¹ represents an unsubstituted hydrocarbon group,
R² represents a substituent,
where, R² in N bonded to L³ represents an unsubstituted hydrocarbon group,
X's each independently represent a group selected from a group G2 of coordinating functional groups below,
L¹, L², and L³ each independently represent a linking group, and
n represents an integer of 1 to 5,
<group G2 of coordinating functional groups>
a carboxy group, a phosphate group, a phosphonate group, a sulfonate group, a sulfinate group, and a hydroxyl group.

6. The metal extractant according to claim 5,
wherein X represents a carboxy group, a phosphate group, a phosphonate group, a sulfonate group, or a sulfinate group.

7. The metal extractant according to claim 1,
wherein the metal extractant is represented by Formula (II), 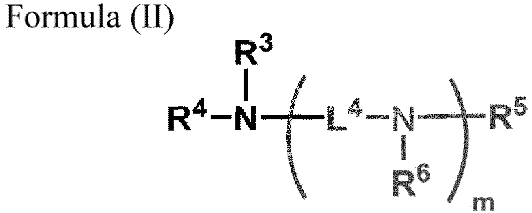
in Formula (II), R³ to R⁵ each independently represent an unsubstituted hydrocarbon group,
R⁶ represents a substituent,
where, R⁶ in N bonded to R⁵ represents an unsubstituted hydrocarbon group,
L⁴ represents a linking group, and
m represents an integer of 1 to 5.

8. A separation recovery method of metal ions, the separation recovery method comprising:
mixing a water phase including plural kinds of metal ions with an oil phase including the metal extractant according to any one of claims 1 to 7.

9. The separation recovery method according to claim 8,
wherein in a case where the oil layer includes the metal extractant according to claim 7, the water phase includes halide ions.

10. The separation recovery method according to claim 8,
wherein metal ions of two or more elements belonging to different groups in a periodic table among plural kinds of metal ions are extracted and recovered from the water phase to the oil phase.

11. The separation recovery method according to claim 10,
wherein the metal ions of the two or more elements are a metal recovery from a waste battery.
